(19) Europäisches Patentamt
European Patent Office
Office européen des brevets

(11) Publication number: **0 465 577 B1**

(12) **EUROPEAN PATENT SPECIFICATION**

(45) Date of publication of patent specification: **16.03.94**

(51) Int. Cl.$^5$: **G01N 33/542**, G01N 33/58, G01N 33/535, G01N 33/532

(21) Application number: **90905997.4**

(22) Date of filing: **28.03.90**

(86) International application number: **PCT/US90/01569**

(87) International publication number: **WO 90/11523 (04.10.90 90/23)**

The file contains technical information submitted after the application was filed and not included in this specification

(54) **CATALYZED REPORTER DEPOSITION.**

(30) Priority: **29.03.89 US 330357**
**20.03.90 US 494226**

(43) Date of publication of application: **15.01.92 Bulletin 92/03**

(45) Publication of the grant of the patent: **16.03.94 Bulletin 94/11**

(84) Designated Contracting States: **AT BE CH DE DK ES FR GB IT LI LU NL SE**

(56) References cited:
**EP-A- 0 144 744**
**WO-A-84/02193**

**JOURNAL OF IMMUNOLOGICAL METHODS, vol. 125, 1989, Elsevier Science Publishers B.V., Amsterdam (NL); M.N. BOBROW et al., pp. 279-285&NUM;**

(73) Proprietor: **E.I. DU PONT DE NEMOURS AND COMPANY**
**1007 Market Street**
**Wilmington Delaware 19898(US)**

(72) Inventor: **BOBROW, Mark, Norman**
**3 Bonnie Way**
**Woburn, MA 01801(US)**
Inventor: **EBERSOLE, Richard, Calvin**
**2412 Dacia Drive**
**Wilmington, DE 19810(US)**
Inventor: **LITT, Gerald, Joseph**
**43 Willow Road**
**Wellesley, MA 02181(US)**
Inventor: **MORAN, John, Richard**
**27 Dogwood Drive**
**Kennett Square, PA 19348(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 465 577 B1**

Rank Xerox (UK) Business Services
(3.10/3.09/3.3.3)

(74) Representative: **von Kreisler, Alek, Dipl.-Chem. et al**
**Patentanwälte**
**von Kreisler-Selting-Werner**
**Postfach 10 22 41**
**D-50462 Köln (DE)**

## Description

FIELD OF THE INVENTION

This invention relates to assays and, more particularly, to catalyzing reporter deposition via an activated conjugate to amplify the detector signal thereby improving detection and/or quantitation of an analyte in a sample.

BACKGROUND OF THE INVENTION

The introduction of immunodiagnostic assays in the 1960s and 1970s greatly increased the number of analytes amenable to precise and accurate measurement. Radioimmunoassays (RIAs) and immunoradiometric (IRMA) assays utilize radioisotopic labeling of either an antibody or a competing antigen to measure an analyte. Detection systems based on enzymes or fluorescent labels were developed as an alternative to isotopic detection systems. Enzyme based assays proved to be more sensitive, faster, less dependent upon expensive, sophisticated instrumentation.

The need for diagnostic assays having simpler formats, increased sensitivity with less dependence upon sophisticated and expensive instrumentation prompted investigators to try to harness the catalytic power of enzymes to develop these newer assays.

D. L. Bates, Trends in Biotechnology, pages 204-209, Vol. 5 No. 7 (1987), describes diagnostics which use a method of enzyme amplification to develop more sensitive and simple immunoassays. In this method a second enzyme system is coupled to the primary enzyme label, e.g., the primary enzyme can be linked catalytically to an additional system such as a substrate cycle or an enzyme cascade. Thus, the essence of enzyme amplification according to Bates is the coupling of catalytic processes wherein an enzyme is modulated by the action of a second enzyme, either by direct modification or by interaction with the product of the controlling enzyme.

U.S. Patent 4, 668, 621, issued to Doellgast on May 26, 1987, describes application of an enzyme-linked coagulation assay (ELCA) to develop an amplified immunoassay using the clotting cascade to enhance sensitivity of detection of immune complexes. The process involves clot formation due to thrombin activated fibrin formation from insolubilized fibrinogen and labeled solubilized fibrinogen. Amplification of the amount of reportable ligand attached to solid phase is obtained only by combining use of clotting factor conjugates with subsequent coagulation cascade reactions. One of the disadvantages of this system is that it can only be used to measure the presence of materials which modulate the activity of one or more of the blood clotting factors. Another disadvantage is that the primary enzyme, thrombin, cannot be immobilized or coupled to a reporter or a member of a specific binding pair.

U.S. Patent 4,463,090, issued to Harris on July 31, 1984, describes a cascade amplification immunoassay requiring a combination of at least two sequential catalyses wherein a first enzyme activates a second enzyme which in turn acts upon the substrate.

Another amplification system is described in U.S. Patent 4,598,042, issued to Self on July 1, 1986, and U.K. Patent Application No. 2,059,421 which was published on April 23, 1981, which disclose an immunoassay using an enzyme label to, produce directly or indirectly a substance that is capable of influencing a catalytic event without itself being consumed during the catalytic event. More specifically, a primary enzyme system produces or removes a substance capable of modulating a secondary enzyme system which results in amplification. The enzyme systems use unconjugated enzymes to avoid the tendency to inactivate certain enzymes on conjugation.

European Patent Application Publication No. 123,265 which was published on October 31, 1984, describes another cascade amplification immunoassay wherein a zymogen-derived-enzyme is coupled to a zymogen-to-enzyme cascade reaction sequence to obtain multiple stages of amplification in producing detectable marker material used to quantify analyte amount.

European Patent Application Publication No. 144,744, published June 19, 1985, describes a specific binding assay based on enzyme cascade amplification wherein the label component employed in the detectant reagent is a participant in or a modulator of an enzyme cascade reaction wherein a first enzyme acts on a first substrate to product a second enzyme. The production of the second enzyme can be followed or the second enzyme can act on a second substrate to produce a third enzyme.

Similarly, U.S. Patent 4,318,980, issued to Boguslaski et al. on March 9, 1982, describes a heterogenous specific binding assay using a conjugate formed of a specific binding substance coupled to the reactant, i.e., an enzymatic reactant. The ability of the reactant to participate in the monitoring reaction to detect the presence of analyte is altered by the presence of the ligand in the medium. Thus, the conjugate

in its free state is more active in the monitoring reaction than in its bound state.

A heterogenous specific binding assay using enzyme amplification is described in British Patent Application No. 1,401,297 which was published on July 30, 1975 and U.S. Patent 4,376,825, issued to Rubenstein et al. on March 15, 1983. Amplification is achieved by bonding the compound to be assayed or a counterfeit of it to an enzyme. The resulting enzyme-bound-ligand competes with free ligand for specific receptor sites. When the enzyme-bound ligand is displaced by the free ligand the enzyme is then free to react with a large number number of substrate molecules and the concentration of the remaining substrate or of the product can be measured. PCT International Publication No. WO 81/00725 which was published on March 19, 1981 describes a method of determining a substrate in a sample which comprises converting the substrate to a product in a first stage of a cyclic reaction sequence and converting the product back to the substrate in a second reaction stage of the cyclic reaction sequence. At least one of the first and second reaction stages is enzyme catalyzed.

PCT Application having International Publication Number WO 84/02193, which was published on June 7, 1984, describes a chromgenic support immunoassay wherein the analyte is contacted with an enzyme-labeled antibody and in which the signal generated by the reaction of the enzyme with its substrate is concentrated on an active support.

European Patent Application Publication No. 181,762, published on May 21, 1986, describes a method to determine enzymatic activity in a liquid sample by particle agglutination or inhibition of particle agglutination.

Substrate/cofactor cycling is another example of amplification which is based on the cycling of a cofactor or substrate which is generated by the primary enzyme label. The primary enzyme converts the primary substrate to an active form which can be cycled by two enzymes of the amplifier cycle. These two enzymes are provided in high concentration and are poised to turn over high concentrations of substrate but are prevented from so doing until the cycling substrate is formed. The product of the primary enzyme is a catalytic activator of the amplifier cycle which responds in proportion to the concentration of substrate and hence the concentration of the enzyme label.

In the early sixties, Lowry et al., Journal of Biological Chemistry, pages 2746-2755, Vol. 236, No. 10 (October 1961), described the measurement of pyridine nucleotides by enzymatic cycling in which the coenzyme to be determined was made to amplify an enzymatic dismutation between two substrates.

A more complex substrate cycling system is described in U.S. Patent 4,745,054, issued to Rabin et al. on May 17, 1988. The Rabin system involves using a small enzymically inactive peptide fragment of an enzyme as a label and conjugated with the complementary fragment to form an enzyme which catalyzes a primary reaction whose product is, or leads to, an essential coenzyme or prosthetic group for a second enzyme which catalyzes a secondary reaction leading to a detectable result indicating the presence of analyte.

Vary et al., Clinical Chemistry, pages 1696-1701, Vol. 32 (1986) describe an amplification method suited to nucleic acids. This is the strand displacement assay which uses the unique ability of a polynucleotide to act as a substrate label which can be released by a phosphorylase.

## SUMMARY OF THE INVENTION

The present invention concerns a method to catalyze reporter deposition to improve detection or quantitation of an analyte in a sample by amplifying the detector signal which comprises immobilizing an analyte dependent enzyme activation system which catalyzes deposition of reporter by activating a conjugate consisting of a detectably labeled substrate specific for the enzyme system, said conjugate reacts with the analyte dependent enzyme activation system to produce an activated conjugate which deposits substantially wherever receptor for the activated conjugate is immobilized, said receptor not being reactive with the analyte dependent enzyme activation system.

In another embodiment the invention concerns an assay for detecting or quantitating the presence or absence of an analyte in a sample using catalyzed reporter deposition to amplify the reporter signal.

This invention also concerns 6-(phenoxy-(4'-azo-2''-carboxyethylphenyl))-hexanoyl-alkaline phosphatase.

## BRIEF DESCRIPTION OF FIGURES

Figure 1 is a graph comparing results of an HSV antigen assay run with and without catalyzed reporter deposition.

Figure 2 is a graph comparing results of an HIV p24 core antigen assay using conjugate concentrations of 0.2, 0.4, and 0.8 µl/ml (Amp 1, 2, and 3, respectively) "HRP" represents a non-amplified assay wherein

the detector antibody was directly labeled with HRP. "Biotin" indicates another non-amplified assay wherein the detector antibody was conjugated to biotin and detected with HRP labeled streptavidin.

Figure 3 is a graph of a mouse IgG assay run using an HRP ADEAS to catalyze deposition of biotin-tyramine which was detected with streptavidin-HRP (HRP-Amp HRP) or with streptavidin-AP (HRP-Amp AP). The assay was also run using only HRP labeled detector antibody or AP labeled detector antibody.

Figure 4 presents two graphs comparing results obtained from a Du Pont HIV p24 antigen ELISA run with and without using catalyzed reporter deposition to amplify reporter signal.

Figure 5 is a graph comparing results of a mouse IgG assay without catalyzed reporter deposition (HRP) and with catalyzed reporter deposition (HRP-$\beta$-Gal).

Figure 6 depicts a preparation of 6-(phenoxy-(4'-azo-2''-carboxyethylphenyl))-hexanoyl-alkaline phosphatase (HEE-6-AP), the synthesis of which is described in Example 10.

Figure 7 illustrates the esterase catalyzed conversion of HEE to 2-(4'-hydroxyphenylazo)benzoic acid (HABA)/streptavidin complex.

## DETAILED DESCRIPTION OF THE INVENTION

The term analyte dependent enzyme activation system (ADEAS) refers to an enzyme system wherein (i) at least one enzyme is coupled, in any manner known to those skilled in the art, to a member of a specific binding pair, or (ii) the enzyme need not be coupled to a member of a specific binding pair when it is the analyte. The enzyme, either by itself or in connection with a second enzyme, catalyzes the formation of an activated conjugate which then is deposited wherever a receptor for the activated conjugate is immobilized.

The term amplification as used herein means amplification of reporter signal due to deposition of a conjugate activated by an ADEAS.

The term conjugate means a detectably labeled substrate specific for the ADEAS whether it be a single enzyme ADEAS or multi-enzyme ADEAS. The substrate must have at least one component but is not limited to such. For example, the substrate can consist of two components. One component contains the binding site for the receptor and is detectably labeled. The other component is a constituent which prevents or interferes with binding to the receptor until such time as the ADEAS primes the conjugate as is discussed below. Another example of a conjugate is biotin-tyramine wherein tyramine is the substrate portion and biotin constitutes the detectable label as described below. Conjugates are described in greater detail below as well.

The term detectably labeled means that the substrate can be coupled to either a reporter or to an unlabeled first member of a specific binding pair provided that the reporter introduces a different moiety to the substrate as is discussed below. When the substrate is coupled to an unlabeled member of a specific binding pair, following deposition, the substrate-specific binding partner complex is reacted with the second member of the binding pair which is coupled to a reporter. Alternately, the substrate-specific binding partner complex can be pre-reacted with the detectably labeled other member of the specific binding pair prior to deposition.

The term deposition means directed binding of an activated conjugate to the receptor which results from either the formation of a covalent bond or a specific binding pair interaction as described below.

The term receptor means a site which will bind to the activated conjugate either through the formation of a covalent bond or a specific binding pair interaction as described below.

The term activated conjugate means that the conjugate has been primed by the ADEAS to bind with the receptor.

One of the unique features of this invention is the analyte dependent enzyme activation system which catalyzes deposition of conjugate by converting the substrate portion of the conjugate to an activated form which is deposited wherever a specific receptor for the activated conjugate is immobilized. The ADEAS does not utilize enzyme cascade reactions or enzyme cycling to effect amplification. Rather, it uses either a single enzyme or combination of enzymes to activate the conjugate. Deposition of conjugate occurs only if the analyte and analyte dependent enzyme activation system, which can be the same if the analyte is an enzyme, for example in the detection of an enzyme such as alkaline phosphatase, or different, have been immobilized and a receptor, as described below, is immobilized to bind the activated conjugate. Thus, the ADEAS, conjugate, and receptor are chosen to form an operational trio.

The following is one embodiment of a single enzyme ADEAS system applied to a forward sandwich immunoassay format: the test sample containing the analyte is reacted with an immobilized capture reagent, such as an antibody; excess reagents are washed off; the immobilized capture antibody-analyte complex is reacted with an ADEAS, such as a second antibody specific for the analyte which has been coupled to an

enzyme, e.g. horseradish peroxidase (HRP), alkaline phosphatase (AP), etc. The ADEAS will bind only if the analyte has been bound by the capture reagent. Otherwise the reagents will be washed off. Coupling of the enzyme to a specific binding partner does not affect the enzyme's ability to react with the substrate portion of the conjugate. When conjugate such as biotin-tyramine or HABA-tyramine analog (e.g., N-(4'''-hydroxyphenethyl)-6-(phenoxy-(4'-azo-2''-benzoic acid))hexamide) is added to the immobilized capture antibody-analyte-second antibody-enzyme complex, the enzyme reacts with the substrate portion of the conjugate, e.g., with the tyramine portion of the conjugate, converting it to an active form which will bind to an immobilized receptor which is either endogenous or exogenous to the assay system. The amount of conjugate deposited will be a function of immobilized ADEAS. Deposited conjugate such as biotin-tyramine or HABA tyramine analog can then be detected by reacting with streptavidin-HRP and orthophenylenediamine. The term HABA-tyramine analog means, generally, unsubstituted or substituted HABA, coupled with or without a spacer, to a hydroxy-phenyl containing compound such as tyramine. If the conjugate is fluorescein-tyramine then the deposited conjugate can be detected directly, or following reaction with a labeled anti-fluorescein antibody.

Thus, the ADEAS is used to catalyze the deposition of detectably labeled substrate (the conjugate) to generate additional signal. The ADEAS is detected directly as part of the overall signal when the enzyme component of the ADEAS is the same as the enzyme used as the reporter. Figure 3 illustrates this situation as well as the situation where an ADEAS enzyme component and reporter enzyme are different and thus, the ADEAS enzyme component is not detected directly as part of the overall signal.

A multi-enzyme ADEAS immunoassay format would involve a similar approach. In the example above, the ADEAS can be an antibody coupled to an enzyme such as AP. In addition to the immobilized capture antibody-analyte-second antibody-AP complex, a second enzyme such as HRP could be immobilized on the support. The conjugate can be a detectably labeled phenylphosphate which cannot react with HRP until it is dephosphorylated. AP dephosphorylates the phenol which then is free to react with the immobilized HRP to form an activated phenolic conjugate which deposits wherever receptors are immobilized. After removing excess reagent, deposited reporter is detected and quantitated. Alternatively, HRP can be coupled to the second antibody and AP can be immobilized on the surface of the support.

The instant invention is surprising and unexpected because amplification of reporter signal is obtained via deposited activated conjugate without using cascade mechanisms or enzyme cycling. The ADEAS reacts with the conjugate to form an activated conjugate which will bind with immobilized receptor specific for the activated conjugate. The amounts of receptor and activated conjugate are in excess of the amount of ADEAS immobilized.

The choice of an ADEAS is governed by the ability of the enzyme or enzymes to convert a conjugate to an activated form which will bind to an immobilized receptor whether endogenous or exogenous. Accordingly, a detailed knowledge of catalytic properties of each specific enzyme is needed in order to properly design the substrate and receptor. Other important factors include availability of the enzyme or enzymes, relative ease or difficulty to couple it to the member of a specific binding pair, stability of the enzyme or enzymes as well as the stability of the conjugate and the receptor. In some cases, an ADEAS can be purchased, depending on the assay format.

Enzymes suitable for use in an ADEAS include hydrolases, lyases, oxidoreductases, transferases isomerases and ligases. There can be mentioned peroxidase, glucose oxidase, phosphatase, esterase and glycosidase. Specific examples include alkaline phosphatase, lipases, beta-galactosidase and horseradish peroxidase,.

Members of specific binding pairs suitable for use in practicing the invention can be of the immune or non-immune type. Immune specific binding pairs are exemplified by antigen/antibody systems or hapten/antihapten systems. The antibody member, whether polyclonal, monoclonal or an immunoreactive fragment thereof, of the binding pair can be produced by customary methods familiar to those skilled in the art. The terms immunoreactive antibody fragment or immunoreactive fragment mean fragments which contain the binding region of the antibody. Such fragments may be Fab-type fragments which are defined as fragments devoid of the Fc portion, e.g., Fab, Fab' and $F(ab')_2$ fragments, or may be so-called "half-molecule" fragments obtained by reductive cleavage of the disulfide bonds connecting the heavy chain components of the intact antibody. If the antigen member of the specific binding pair is not immunogenic, e.g., a hapten, it can be covalently coupled to a carrier protein to render it immunogenic.

Non-immune binding pairs include systems wherein the two components share a natural affinity for each other but are not antibodies. Exemplary non-immune binding pairs are biotin-avidin or biotin-streptavidin, folic acid-folate binding protein, complementary probe nucleic acids, etc. Also included are non-immune binding pairs which form a covalent bond with each other but are not antibodies. Exemplary covalent binding pairs include sulfhydryl reactive groups such as maleimides and haloacetyl derivatives and

amine reactive groups such as isothiocyanates, succinimidyl esters and sulfonyl halides, etc.

Suitable supports used in assays include synthetic polymer supports, such as polystyrene, polypropylene, substituted polystyrene, e.g., aminated or carboxylated polystyrene; polyacrylamides; polyamides; polyvinylchloride, etc.; glass beads; agarose; nitrocellulose, etc.

Another important component of the invention is the conjugate, i.e., a detectably labeled substrate which must be specific for the ADEAS. As was stated above, when the conjugate reacts with the ADEAS, the enzyme or enzymes catalyze formation of an activated conjugate which binds wherever a receptor is immobilized whether exogenous or endogenous. An immobilized exogenous receptor means a receptor which does not originate within the assay. It must be immobilized on the surface of the support prior to adding the conjugate to the reaction mixture. An endogenous receptor means a receptor which originates within the assay and does not require immobilization prior to adding the conjugate because the receptor is immobilized within the assay system.

For example, when an HRP ADEAS (HRP coupled to a member of a specific binding pair) is reacted with conjugate containing a phenolic substrate, an activated phenolic substrate is produced. It is believed that the activated phenolic substrate binds to electron rich moieties such as tyrosine and tryptophan present in the proteins on the solid support. However, if a different conjugate is used, such as a labeled 3-methyl-2-benzothiazolinone hydrazine (MBTH) which is discussed below, a receptor, such as 3-(dimethylamino)-benzoic acid (DMAB), must be immobilized prior to addition of conjugate.

Another embodiment involves reacting a conjugate which becomes phosphorylated by an ADEAS. The activated (phosphorylated) conjugated can then react with an antibody specific for the activated conjugate.

In still another variation, an ADEAS can be reacted with a conjugate consisting of a component which when activated will bind to a receptor and which is coupled to a component having a thiol reactive group such as a maleimide. The deposited maleimide moiety can then be detected by reacting with a sulfhydryl-containing reporter which can be endogenous to the reporter, e.g., beta-galactosidase, or the sulfhydryl groups can be added to reporters such as HRP or AP using thiolating reagents such as N-succinimidyl-S-acetylthioacetate (SATA), S-acetylmercaptosuccinic anhydride (SAMSA), or succinimidyl-3-(acetylthio)-propionate (SATP).

Alternatively, the substrate can be coupled to a protected sulfhydryl containing group and this can be used as the conjugate. After binding to the receptor, this can be deprotected using conventional techniques known to those skilled in the art. Detection can be effected using a reporter having a thiol reactive group such as maleimide-HRP or iodoacetyl-HRP.

Another alternative is to use a conjugate wherein the substrate has two components as described above, a detectably labeled first component which will bind to the receptor after the second component has been activated or removed by the ADEAS. An example of this is a small organic molecule such as 2-(4'-hydroxy-phenylazo)-benzoic acid (HABA) which binds specifically to avidin and streptavidin. The terms avidin and streptavidin are used herein interchangeably. HABA can be detectably labeled using any of the reporters described below, e.g., radioisotopes, enzymes, etc. For instance, alkaline phosphatase (AP) can be conjugated to HABA, using techniques well known to those skilled in the art, with or without a spacer, to a functional group on HABA. An example of such a functional group is the 4'-hydroxyl moiety. Moreover, HABA can be modified to possess a second component which prevents binding until it has been removed by the ADEAs. For example, esterification of HABA with ethanol produces a HABA ethyl ester which does not bind to streptavidin. Detectably labeled HABA ethyl esters will not bind to streptavidin until the ester group has been hydrolyzed to the corresponding carboxylic acid. Hydrolysis can be affected using an enzyme such as an esterase, e.g., porcine liver esterase. Thus, detectably labeled HABA esters such as 6-(phenoxy-(4'-azo-2''-carboxyethylphenyl))-hexanoyl-alkaline phosphatase can be deposited using an ADEAS having a suitable esterase which will hydrolyze the ester to permit binding of detectably labeled HABA with streptavidin (i.e., exogenous receptor) which has been immobilized on the surface of a support.

Compounds of the formula

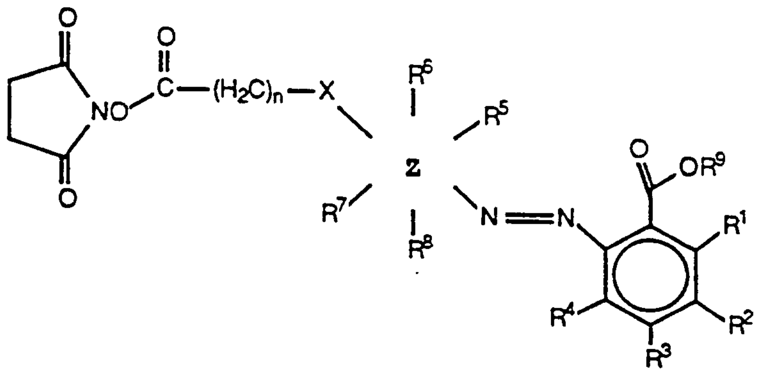

wherein

$R^1$ through $R^8$ are the same or different and are selected from the group consisting of straight chain or branched alkyl groups having 1-4 carbon atoms, F, Cl, Br or I;

Z is phenyl or naphthyl;

n is 1-19;

X is N, O, S; and

$R^9$ can be H or straight chain or branched alkyl group having 1-4 carbon atoms can be used to synthesize HABA-type conjugates as described in the examples below. The term HABA-type conjugate means HABA derivatives: (i) which can be substituted or unsubstituted and are coupled with a spacer to a reporter and (ii) which contain an ADEAS activatable moiety that prevents the conjugate from binding to streptavidin until it has been activated or removed by the ADEAS. The synthesis of such a compound is illustrated in Figure 6 as well in Example 10 below. The approach described below can be modified by those skilled in the art generally to synthesize any of these compounds using procedures well known to those skilled in the art.

Other small organic molecule/receptor combinations which are suitable to practice the invention include haptens/antibodies, sugars and oligosaccharides/lectins, biotin and dyes/avidin and/or streptavidin.

As is shown in Table 1, a number of receptors are available. The choice of a receptor will depend upon the conjugate selected.

The optimal concentration of conjugate is determined according to the procedure explained in Example 1. Optimal concentrations will vary depending upon enzyme used in the ADEAS and substrate selected to produce conjugate.

Conjugate can be synthesized using conventional coupling and labeling techniques. Substrate choice will depend upon the ADEAS selected. To reiterate, detailed knowledge is required of the catalytic properties of each specific enzyme in order to properly design a useful synthetic substrate and, if necessary, a receptor.

A wide variety of reporters are available for coupling to the substrate to produce the conjugate or to couple to a member of a specific binding pair. As was discussed above reporter should introduce a different moiety to the substrate. Reporters can be a radioactive isotope, such as, $^{125}I$, enzymes, fluorogenic, chemiluminescent, electrochemical or magnetic materials. Internally labeled reporters (e.g., tritium or other such radionuclides) which do not introduce a different moiety to the substrate are not contemplated for practicing the invention.

Examples of reporter enzymes which can be used to practice the invention include hydrolases, lyases, oxidoreductases, transferases, isomerases and ligases some preferred examples are phosphatases, esterases, glycosidases and peroxidases. There can be mentioned peroxidase, glucose oxidase, phosphatase, esterase and glycosidase. Specific examples include alkaline phosphotase, lipases, beta-galactosidase and horseradish peroxidase. As was noted above, if an enzyme is used as a reporter, it can be the same as or different from the enzyme or enzymes used in the ADEAS. The instant invention can be used to catalyze deposition of a radioisotopically labeled conjugate or an enzyme-labeled conjugate, etc.

Another embodiment of the forward sandwich immunoassay described above would involve reacting a capture-antibody-analyte-second antibody complex with an ADEAS consisting of an anti-antibody coupled to an enzyme such as HRP or AP. The anti-antibody would bind an epitope on the second antibody.

This invention is not limited to sandwich immunoassays. It is applicable to a wide variety of assay formats, for example, nucleic acid hybridization assays for both RNA and DNA.

To further illustrate the invention, examples of single and multi-enzyme ADEAS', conjugates, receptors, and receptor types are presented in Table 1 below.

TABLE 1

| ADEAS | | CONJUGATE[3] | RECEPTOR | |
|---|---|---|---|---|
| Class | Enzyme | Substrate | Class | Type |
| Single Enzyme | HRP[1] | Substituted phenols, e.g., tyramine | Endogenous | Phenols; electron rich moieties |
| Single Enzyme | HRP[1] | MBTH | Exogenous | DMAB |
| Single Enzyme | β-Galactosidase | β-Galactopyranosyl-glycoside, e.g., of fluorescein | Exogenous | Antibody to deglycosylated moiety, e.g., anti-fluorescein |
| Single Enzyme | AP | NADP | Exogenous | NAD binding proteins |
| Single Enzyme | AP | Substituted phosphates, e.g., nitrophenyl phosphate | Exogenous | Antibody to dephosphorylated product, e.g., anti-nitro-phenol |
| Single Enzyme | AP | Phosphorylated biotin | Exogenous | Avidin; streptavidin |
| Multi-Enzyme | AP or HRP[1,2] | Phosphorylated substituted phenols, e.g., tyrosine phosphate | Endogenous | Phenols; electron rich moieties |
| Multi-Enzyme | HRP[1] or β-galactosidase[2] | β-galatopyranosyl-glycoside of a phenolic compound, e.g., nitro-phenyl-galactoside | Endogenous | Phenols; electron rich moieties |

1 HRP requires the presence of $H_2O_2$.

2 Does not matter which enzyme is coupled to member of specific binding pair.

3 Label can be a reporter or member of a specific binding pair.

In the AP/HRP multi-enzyme ADEAS described above, the conjugate must be dephosphorylated before it will react with HRP; and in the $\beta$-gal/HRP multi-enzyme ADEAS, the conjugate must be deglycosylated before it will react with HRP.

It should be clear to those skilled in the art that a large number of variations are possible and all these variations fall within the scope of the invention.

The following examples are intended to illustrate the invention. Unless otherwise indicated, 100 µl of all reagents were used. The one exception was that 200 µl of blocking buffer was used.

EXAMPLE 1

Preparation of Conjugates and Optimization Of Conjugate Concentration

Para-hydroxyphenylpropionyl biocytin (HPPB) was prepared by mixing a solution of p-hydroxyphenylpropionic acid-N-hydroxysuccinimide ester (50 mg [0.2 mMol]/2 ml dimethyl sulfoxide) with biocytin (70.75 mg [0.2 mMol]/2 ml 0.1 M $NaHCO_3$) overnight at room temperature (RT). Biotin-tyramine (BT) was prepared by mixing a solution of tyramine (40 mg [0.3 mMol]/1 ml dimethyl sulfoxide) with biotin-N-hydroxysuccinimide ester (100 mg [0.3 mMol]/1 ml dimethyl sulfoxide) overnight at RT. The solutions of HPPB and BT were used as is. The calculated concentrations were 26 mg/ml for HPPB and 55 mg/ml for BT.

Polystyrene EIA strips (NUNC) were coated with polyclonal anti-Herpes Simplex Virus (HSV) antibody (Dako, Carpenteria, CA) in 0.1 M carbonate buffer pH 9.6 overnight at 4°C, and then blocked with 2% bovine serum albumin (BSA) in carbonate buffer and then washed with 10 mM phosphate buffered saline, 0.05% Tween® 20, pH 7.4 (PBST). A dilution of HSV antigen in 1% BSA, 10 mM phosphate buffered saline, 0.05% Tween® 20 pH 7.4 (BSA-PBST), or buffer without antigen, was incubated for 1 hour at 37°C. The dilution was sufficient to obtain the optical densities in the range reported in Table 1. It was washed with PBST. The analyte dependent enzyme activation system consisted of HRP coupled to anti-HSV (HRP ADEAS) which was purchased from Dako. The HRP ADEAS was added and incubated for 30 min. at RT and was washed with PBST. Various concentrations of HPPB or BT as set forth in Table 2 below, were added in 50 mM tris-HCl, 0.01% $H_2O_2$, pH 8.0, for 15 min. at RT. After washing with PBST, streptavidin-HRP was added and incubated for 15 min. at RT to react with deposited biotins. The plate was then washed with PBST. An HRP substrate, o-phenylenediamine (OPD), was added, incubated for 30 min. at RT, and stopped with 4 N $H_2SO_4$. Optical densities at 490 nm were recorded on a microtiter plate reader.

Results

Results are presented in Table 2. Column 1 presents the various concentrations in µl/ml of HPPB or BT. Columns 2 and 3 present the optical densities recorded as a function of HPPB concentration. Columns 4 and 5 present the results obtained using BT.

HPPB and BT were converted to activated forms by HRP ADEAS. Catalyzed reporter deposition was achieved without immobilizing a receptor.

In choosing the optimal concentration, one must look at both the magnitude of signal amplification as well as the signal to noise ratio. With this in mind, the optimal concentration of HPPB was 20 µl/ml (approximately 0.5 mg/ml), and that of BT, was about 0.3 µl/ml (approximately 16 µg/ml).

TABLE 2

| Absorbance 490 nm | | | | |
|---|---|---|---|---|
| Conc. HPPB or BT (µl/ml) | HPPB CONJUGATE | | BT CONJUGATE | |
| | HSV | Buffer (w/o Ag)* | HSV | Buffer (w/o Ag) |
| 0 | 0.079 | 0.031 | 0.079 | 0.031 |
| 20 | 1.155 | 0.181 | 0.700 | 0.165 |
| 10 | 0.904 | 0.140 | --- | --- |
| 5 | 0.499 | 0.120 | 2.060 | 0.430 |
| 2.5 | 0.177 | 0.063 | --- | --- |
| 1.25 | 0.113 | 0.062 | 2.230 | 0.502 |
| 0.625 | 0.103 | 0.048 | --- | --- |
| 0.313 | --- | --- | 1.880 | 0.169 |
| 0.078 | --- | --- | 0.263 | 0.051 |
| 0.020 | --- | --- | 0.090 | 0.040 |

* w/o Ag = without antigen

EXAMPLE 2

Amplification of Detector Signal In HSV Assay Using Catalyzed Reporter Deposition

Anti-HSV coated EIA strips were prepared as described in Example 1. A 1:100 dilution of HSV antigen was prepared and serially four-fold diluted. These dilutions of HSV were incubated for 2 hours at 37°C with the anti-HSV coated EIA strips. Excess reagent was washed off with PBST. The ADEAS was the same as that described in Example 1 above. It was added to the anti-HSV coated EIA strips containing the anti-HSV-HSV complex and incubated for 30 min. at RT and then washed with PBST. 20 µl/ml of HPPB conjugate as determined in Example 1 was added in 50 mM tris-HCl, 0.01% $H_2O_2$, pH 8.0, and was incubated for 15 min. at RT and then washed with PBST. Deposited biotins were reacted with streptavidin-HRP (SA-HRP) for 15 min. at RT. It was washed with PBST. The substrate, OPD, was added and incubated 30 min. at RT, stopped with 4 N $H_2SO_4$, and the absorbance at 490 nm was recorded on a microtiter plate reader.

Non-amplified assays were run in which (a) no HPPB and no SA-HRP were used; (b) HPPB was used without SA-HRP; (c) SA-HRP was used without HPPB.

Results

The results shown in Figure 1 demonstrate that

(a) catalyzed deposition of reporter was obtained and

(b) both the conjugate and SA-HRP were needed for detection because the conjugate contained an unlabeled member of a specific binding pair.

Results for the non-amplified assay (no HPPB, no SA-HRP) were plotted. The results for the other assays were not plotted because the additional plots would overlap with the non-amplified results already plotted.

EXAMPLE 3

Amplification of Detector Signal in HIV p24 Assay Using Catalyzed Reporter Deposition:

Effect of Conjugate Concentration

Polystyrene EIA strips (NUNC) were coated with rabbit anti-HIV p24 antibodies in 0.1 M carbonate buffer, pH 9.6, overnight at 4°C, and then blocked with 2% BSA in carbonate buffer followed by washing with PBST. HIV antigen was incubated for 2 hours at 37°C (concentrations are indicated in Figure 2). The plate was then washed with PBST. A rabbit anti-HIV p24-HRP analyte dependent enzyme activation system was then incubated for 2 hours at 37°C, and washed with PBST. Various concentrations of BT conjugate,

(0.2, 0.4, and 0.8 µl/ml) in 0.1 M borate buffer, 0.01% $H_2O_2$, pH 8.5, were incubated for 15 min. at RT followed by washing with PBST. Then streptavidin-HRP was incubated for 15 min. at RT.

As a comparison, a biotinylated anti-HIV p24 antibody was used, and detected with streptavidin-HRP. OPD was added and incubated for 30 minutes, stopped with 4 N $H_2SO_4$, and optical densities at 490 nm were recorded on a microtiter plate reader.

Results

The results are shown in Figure 2 where Amp 1, Amp 2, and Amp 3 refer to BT at concentrations of 0.2, 0.4, and 0.8 µl/ml respectively. Different levels of amplification were achieved using catalyzed reporter deposition depending on the concentration of conjugate.

Figure 2 also presents results for a non-amplified assay using a biotinylated antibody/SA-HRP detection system (biotin) and a non-amplified assay wherein anti-HIV p24 detector was directly labeled with HRP. The results obtained using the anti-HIV p24-HRP detector were inferior compared to the significant increase in detector signal obtained using catalyzed reporter deposition.

Depending upon the concentration of conjugate, signals as good, and greater, as those obtained with the biotinylated antibody were obtained using the catalyzed reporter deposition method of the instant invention. Best results were obtained using conjugate concentration near the optimal amount as was determined in Example 1.

EXAMPLE 4

Preparation and Characterization of Biotin Tyramine

Preparation of biotin-tyramine: a solution of biotin-N-hydroxysuccinimide, 170 mg (0.5 mMoles), and tyramine (recrystallized from water), 68.5 mg (0.5 mMoles), in 25 ml dimethylformamide was treated with 10 ml of 1 M triethylammonium bicarbonate, pH 7.5, and then heated at 50 °C for 3 hours.

Isolation: the solution was concentrated to dryness on a rotary evaporator, and the residue was recrystallized from water, with a yield of 72%.

Characterization: the melting point was determined to be 192-193 °C.

EXAMPLE 5

Amplification of Detector Signal In A Mouse IgG Assay Using Catalyzed Reporter Deposition

Polystyrene EIA strips (NUNC) were coated with goat anti-mouse IgG (Fc fragment specific) antibody (ICN) in 0.1 M carbonate buffer pH 9.6, overnight at RT. They were then blocked with 2% BSA in carbonate buffer and washed with PBST. Dilutions of mouse IgG in BSA-PBST were incubated in the wells for 1 hour at 37 °C followed by washing with PBST. Concentrations are set forth in Figure 3. Goat anti-mouse IgG-HRP (HRP ADEAS) and goat anti-mouse IgG-Alkaline Phosphatase (AP ADEAS) (Boehringer Mannheim) were diluted as recommended by the manufacturer and incubated for 1 hour at 37 °C. Assays were run with and without catalyzed reporter deposition. The AP ADEAS was not used to catalyze reporter deposition in this experiment.

For catalyzed reporter deposition using the HRP ADEAS, a 1 mg/ml stock solution of biotin tyramine (as described in Example 4) in dimethyl sulfoxide was prepared, and then added to a 0.1 M borate buffer, pH 8.5, 0.01% $H_2O_2$, at 10 µl/ml (10 µg/ml biotin tyramine) and incubated for 15 min. at RT. The plate was then washed with PBST. Streptavidin-HRP (for HRP-Amp HRP), or streptavidin-Alkaline Phosphatase (for HRP-Amp Alk Phos) were incubated for 15 min. at RT and the plate was washed with PBST. Spectrophotometric detection was achieved after the addition of OPD (for HRP), or p-nitrophenyl phosphate (for AP) for 15 min. at RT. Reactions were stopped by the addition of 4 N $H_2SO_4$ (HRP/OPD), or 1 N NaOH (Alk Phos/pNPP). Optical densities, at 490 nm for HRP/OPD and 405 nm for Alk Phos/pNPP, were recorded on a microtiter plate reader.

Results

The results are shown in Figure 3. As is apparent, one can achieve signal amplification with a concomitant lower detection limit by allowing the HRP ADEAS to catalyze deposition of an activated BT conjugate followed by detection with streptavidin coupled to HRP or AP. This example shows that if the

reporter is an enzyme, it can be the same as, or different from, the enzyme used in the ADEAS.

EXAMPLE 6

Amplification Of Detector Signal In An Assay For HIV p24 Which Utilizes A Biotinylated Detector Antibody/Streptavidin-HRP Detection System

The Du Pont HIV p24 Antigen ELISA (catalog number NEK 060) was modified for catalyzed reporter deposition as follows: SA-HRP was used at 1/4 the concentration indicated in the directions. This was followed by a 15 min. RT incubation with biotin-tyramine, 10 µg/ml, in 0.1 M borate, 0.01% $H_2O_2$ pH 8.5 buffer (as in Example 5) . Following washing with PBST, SA-HRP at 1/16 the concentration was incubated for 15 min. at RT. Finally, OPD was added as per kit directions. Except for extending the standard concentrations down to 0.39 pg/ml, no other changes were made.

Results

The results are shown in Figure 4. This experiment demonstrated that one can amplify the signal generated by a biotinylated antibody/SA-HRP system using catalyzed reporter deposition. Because the concentration of SA-HRP for both incubations was much less than that for the non-amplified assay, it was clear that the increased signal was attributable to reporter deposition and not to a double SA-HRP incubation.

EXAMPLE 7

Reporter Deposition on Membranes

Nitrocellulose (Schleicher & Schuell, BA 85) was spotted with HSV antigen, and then blocked with 1% BSA, 1% non-fat dry milk, in PBS buffer, overnight. The membranes were incubated for 1 hour at RT with the analyte dependent enzyme activation system described in Example 1 above. The membranes were then incubated with biotin tyramine (from Example 1) at 2 µl/10 ml 50 mM tris-HCl, 0.01% $H_2O_2$, pH 8.0 buffer for 15 min. at RT, which was followed by incubation with streptavidin-alkaline phosphatase for 15 min. at RT. Controls were run where biotin tyramine was incubated without streptavidin-alkaline phosphatase, and streptavidin-alkaline phosphatase was incubated without biotin-tyramine. Visualization of deposited alkaline phosphatase was facilitated by the addition of BCIP/NBT (Kirkegaard & Perry). BCIP is 5-bromo-4-chloro-indoxyl phosphate and NBT is 2,2'-di-(p-nitrophenyl)-5,5'-diphenyl-3,3'-(3,3'-dimethoxy-4,4'-diphenylene)-ditetrazolium chloride. Visualization of the bound anti-HSV-HRP conjugate was facilitated by the addition of diaminobenzidine (DAB).

Results

Addition of DAB produced observable brown spots where HSV antigen was spotted on the nitrocellulose membrane. Addition of BCIP/NBT produced observable blue spots where HSV antigen was spotted when biotin tyramine and streptavidin-alkaline phosphatase were incubated with the membrane. This showed that alkaline phosphatase was deposited due to HRP activation of the biotin tyramine conjugate, followed by streptavidin-alkaline phosphatase detection.

EXAMPLE 8

Deposition of Beta-Galactosidase by Horseradish Peroxidase, and Detection by Fluorescence

Polystyrene EIA strips (NUNC) were coated with goat anti-mouse IgG (Fc fragment specific) antibody (ICN) in 0.1 M carbonate buffer, pH 9.6, overnight at RT. They were then blocked with 2% BSA in carbonate buffer and washed with PBST. Concentrations of mouse IgG in BSA-PBST, as set forth in Figure 5, were incubated for 1 hour at 37°C followed by washing with PBST. Goat anti-mouse IgG-HRP (ADEAS) purchased from Boehringer Mannheim was diluted as recommended by the manufacturer and incubated for 1 hour at 37°C. The plate was then washed with PBST. A 1 mg/ml stock solution of BT conjugate (as described in Example 4) in dimethyl sulfoxide was prepared, and then added to a 0.1 M borate, 0.01% $H_2O_2$, pH 8.5 buffer at 10 µl/ml (10 µg/ml biotin tyramine). The mixture was added to the plate and

incubated for 15 min. at RT, and then washed with PBST. Streptavidin-beta galactosidase (Bethesda Research Labs) was added and incubated for 15 min. at RT. The assay was also run without catalyzed reporter deposition, i.e., without adding BT. Colorimetric detection of the non-amplified assay was achieved after incubation with OPD (for HRP), for 15 min. at RT. Fluorescent detection of the amplified assay was achieved after the addition of 4-methylumbelliferyl beta-D-galactoside (MUG) (for HRP-beta Gal). Optical densities at 490 nm were recorded for HRP/OPD on a microtiter plate reader. Fluorescence for HRP-beta Gal/MUG was recorded on a fluorescence microtiter plate reader (Dynatech Laboratories).

Results

The results are shown in Figure 5. The fluorescent signal was due to the quantitative deposition of biotintyramine by the HRP ADEAS followed by incubation with streptavidin beta-galactosidase.

EXAMPLE 9

Amplification of a Membrane Assay

Fluorescein-tyramine (FT) was prepared as follows: Solutions of 46.6 mg of 5- (and 6)-carboxyfluorescein succinimidyl ester in 0.3 ml dimethyl sulfoxide and 14.6 mg tyramine in 0.3 ml dimethyl sulfoxide were prepared. Conjugation was achieved by mixing 0.25 ml of each solution overnight at RT. The solution was used as is.

Three nitrocellulose (Schleicher & Schuell, BA 83) strips were spotted with 1 µl of mouse IgG at 10 µg/ml, and serial two fold dilutons in PBS. The membranes were blocked with 5% non-fat dry milk in PBST for 30 min, and then washed three times in PBST. A goat anti-mouse IgG-HRP conjugate (Boehringer Mannehim) diluted 1/2000 in 1% BSA-PBST was incubated for 30 min. at RT, and the membrane were washed three times with PBST. The third membrane was incubated with FT at 20 µg/ml in 0.1 M borate, 0.01% $H_2O_2$, pH 8.5 buffer for 15 min. at RT, and washed three times in PBST. Then, the second and third membranes were incubated with an anti-fluorescein antibody (Chemicon) which was conjugated to HRP (by the SMCC method of Ishikawa, E., et al., J. Immunoassay, 4, 209-327, 1983) diluted in 1% BSA-PBST for 15 min at RT, and washed three times in PBST. Visualization of all three strips was facilitated by the addition of diaminobenzidine for 5 min.

Results

Three spots could be seen on the first two strips indicating a detection limit of 2.5 µg/ml, and that the anti-fluorescein-HRP conjugate did not contribute to additional signal. Six spots could been seen on the third strip indicating a detection limit of 313 ng/ml. The use of the catalyzed reporter deposition amplification method of the invention improved the detection limit of the assay eight fold over that of the non-amplified assay.

EXAMPLE 10

Synthesis of the Conjugate, HEE-6-AP, from (N-succinimidyl)-6-(phenoxy-(4'-azo-2''-carboxyethylphenyl))-hexanoate, HEE-6-NHS, and Alkaline Phosphatase (AP)

The following reaction scheme is illustrated in Figure 6: Ethyl 2-(4'-hydroxyphenylazo)benzoate (HEE), is prepared from 2-(4'-hydroxyphenylazo)-benzoic acid (HABA, (1)), anhydrous ethanol and a catalytic amount of acetyl chloride. The ethyl ester (HEE) is reacted with t-butyl 6-iodohexanoate and sodium hydride using the general procedure reported by Castellanos et al., Tetrahedron, pages 1691-1696, Vol. 37, (1981), to produce t-butyl-6-(phenoxy-(4'-azo-2''-carboxyethylphenyl))hexanoate (HEE-6-t-Bu, (3)). The tert-butyl ester is hydrolyzed by treatment with trifluoroacetic acid using the general procedure reported by Bryan et al., Journal of the American Chemical Society, pages 2353-2355, Vol. 99, (1977), to produce 6-(phenoxy-(4'-azo-2''-carboxyethylphenyl)-hexanoic acid (HEE-6-H). (N-succinimidyl)-6-(phenoxy-(4'-azo-2''-carboxyethylphenyl))hexanoate (HEE-6-NHS, (5)) is prepared from HEE-6-H, N-hydroxysuccinimide and dicyclohexylcarbodiimide in THF using the general procedure reported by Bryan et al., Makromolecular Chemie, pages 2375-2382, Vol. 186, (1985). The NHS ester (5) is dissolved in a minimum volume of DMSO and added to a buffered aqueous solution of alkaline phosphatase (e.g. calf intestine) using the general procedure reported by O'Sullivan et al., Methods in Enzymology, pages 147-166, Vol. 73, (1981) to give the

conjugate (HEE-6-AP, (6)).

EXAMPLE 11

Synthesis of HABA-Tyramine Conjugate (H-T) from (N-succinimidyl)-6-(phenoxy-(4'-azo-2''-carboxyethylphenyl))hexanoate (HEE-6-NHS, (5)) and Tyramine

The NHS ester (5) (1 mmol) described in Example 10 above, and tyramine (1 mmol) are dissolved in DMF (5 mℓ) and stirred at RT for 48 hrs. The solution is evaporated to dryness in vacuo. The residue is suspended in $H_2O$ (pH 8.0, 50 mℓ) and porcine liver esterase (100 mg) is added. The pH of the solution is maintained by adding 0.1 N NaOH as required. The solution is evaporated to dryness after 24 hours. The resulting H-T conjugate is isolated by chromatography (silica gel, chloroform/methanol).

EXAMPLE 12

Amplification of Detector Signal In a Mouse IgG Assay Using Porcine Liver Esterase (PLE) Catalyzed Reporter-Enzyme Deposition

Microtiter plate strips (Nunc) are coated with a mixture of goat anti-mouse IgG (Fc fragment specific) antibody (ICN) and streptavidin (Scripps Laboratories) in 0.1M carbonate buffer pH 9.6 overnight at RT. They are then blocked with 2% BSA in PBS and washed with PBST. Dilutions of mouse IgG (0-100 ng/mℓ) in BSA-PBST are incubated in the wells for 1 hr. at 37°C followed by washing with PBST. A preparation of goat anti-mouse IgG-PLE (PLE ADEAS) (0.75 mg/mℓ) is prepared by the general method described by Hashida et al., Journal of Applied Biochemistry Vol. 6, pages 56-63, 1984 and diluted 1:100-1:2000 with phosphate buffer (0;1 M, pH 8.0, 0.2% BSA) and is incubated for 1 hr. at 37°C and washed with PBST. HEE-6-AP (1 mg/mℓ) (prepared as described in Example 10) is added to the microtiter plate well and incubated for at least 15 min. at 37°C. The plate is then washed with PBST. Spectrophotometric detection is achieved after the addition of p-nitrophenyl phosphate. Reactions are stopped by the addition of 1 N NaOH. Optical densities at 405 nm are recorded on the microtiter plate reader. Amplification of detector signal in this example results from catalyzed reporter-enzyme deposition, i.e., PLE catalyzes deposition of HEE-6-AP where the receptor, streptavidin, has been immobilized on the microtiter plate surface.

EXAMPLE 13

Demonstration of the Enzyme Modulated Binding of a Blocked Binder

A suspension of porcine liver esterase was added (10 µl, 2860 U/mℓ, Cat. No. E3128, Sigma Chemical, St. Louis, MO) to a solution of ethyl 2-(4'-hydroxy-phenylazo)benzoate (HEE) (0.25 mM) and streptavidin (0.2 mg/mℓ) in phosphate buffer (0.1 M, pH 8.0, 2mℓ). A second solution identical to the first was prepared which contained no streptavidin. The absorbance of the two solutions was measured at 500 nm as a function of time. Figure 7 shows that the absorbance of the solution which contained no streptavidin decreased over time indicating hydrolysis of HEE to 2-(4'-hydroxy-phenylazo)benzoic acid (HABA). The absorbance of the solution which contained streptavidin increased over time indicating the formation of the HABA:streptavidin complex, which is known to have a strong absorbance at 500 nm.

## Claims

**Claims for the following Contracting States : AT, BE, CH, DE, DK, FR, GB, IT, LI, LU, NL, SE**

**1.** A method for the detection or quantitation of an analyte in an assay which comprises using an analyte dependent enzyme activation system comprising at least one enzyme to react with a conjugate consisting of a detectably labeled substrate specific for the enzyme system to form an activated conjugate which deposits substantially wherever at least one receptor for the activated conjugate is immobilized, said receptor not being reactive with the analyte dependent enzyme activation system, wherein deposited detectable labels either directly or indirectly generate a signal which can be detected or quantitated.

**2.** A method according to claim 1 wherein at least one enzyme of the analyte dependent enzyme activation system is selected from the group consisting of oxidoreductases, hydrolases, lyases,

transferases, isomerases, and ligases.

**3.** A method according to claim 2 wherein the enzyme is selected from the group consisting of peroxidases, oxidases, phosphatases, esterases and glycosidases.

**4.** A method according to claim 3 wherein the enzyme is selected from the group consisting of horseradish peroxidase, glucose oxidase, alkaline phosphatase and beta-galactosidase, and preferably is horseradish peroxidase.

**5.** A method according to claim 4 wherein the conjugate is selected from the group consisting of biotin-tyramine, p-hydroxyphenylpropionylbiocytin, or fluorescein-tyramine.

**6.** A method according to claim 1 wherein the conjugate is reacted with detectably labeled antibody.

**7.** A method according to claim 1 wherein the conjugate is reacted with a detectably labeled member of a specific binding pair.

**8.** A method according to claim 1 wherein the conjugate is reacted with detectably labeled streptavidin.

**9.** A method according to claim 1 wherein the detectable label is selected from the group consisting of enzymes, radioactive isotopes, fluorogenic, chemiluminescent, or electrochemical materials or a member of a specific binding pair.

**10.** A method according to claim 1 wherein the conjugate is 6-(phenoxy-(4'-azo-2''-carboxyethylphenyl))-hexanoyl-alkaline phosphatase.

**11.** An assay for detecting or quantitating the presence or absence of an analyte in a sample which comprises

a) immobilizing the analyte;

b) reacting the product of step (a) with an analyte-dependent enzyme activation system;

c) reacting the product of step (b) with a conjugate consisting of a detectably labeled substrate to form an activated conjugate which deposits substantially wherever receptor for the activated conjugate is immobilized, said receptor not being reactive with the analyte dependent enzyme activation system; and

d) detecting or quantitating the presence or absence of the analyte in the sample.

**12.** An assay according to claim 11 wherein the analyte dependent enzyme activation system has at least one enzyme selected from the group consisting of oxidoreductases, hydrolases, lyases, transferases, isomerases, and ligases.

**13.** An assay according to claim 12 wherein the enzyme is selected from the group consisting of peroxidases, oxidases, phosphatases, esterases and glycosidases.

**14.** An assay according to claim 13 wherein the enzyme is selected from the group consisting of horseradish peroxidase, glucose oxidase, alkaline phosphatase, and beta-galactosidase.

**15.** An assay according to claim 11 wherein the detectable label is selected from the group consisting of enzymes, radioactive isotopes, fluorogenic, chemiluminescent, or electrochemical materials or a member of a specific binding pair.

**16.** An assay according to claim 14 wherein the enzyme is horseradish peroxidase and the conjugate is selected from the group consisting of biotin tyramine, p-hydroxyphenylpropionyl-biocytin or fluorescein-tyramine.

**17.** A method according to claim 11 wherein the conjugate is 6-(phenoxy-(4'-azo-2''-carboxyethylphenyl))-hexanoyl-alkaline phosphatase.

**18.** An assay according to claim 11 wherein the conjugate is reacted with a detectably labeled member of a specific binding pair.

**19.** An assay according to claim 11 wherein the conjugate is reacted with detectably labeled antibody.

**20.** An assay according to claim 11 wherein the conjugate is reacted with detectably labeled streptavidin.

**21.** An assay according to claim 11 wherein said assay is an immunoassay.

**22.** An assay for detecting or quantitating the presence or absence of an analyte in a sample which comprises

a) reacting an analyte dependent enzyme activation system with a conjugate consisting of a detectably labeled substrate to form an activated conjugate which deposits substantially wherever receptor for the activated conjugate is immobilized, said receptor not being reactive with the analyte dependent enzyme activation system; and

b) detecting or quantitating the presence or absence of the analyte in the sample.

**23.** An assay according to claim 22 wherein the analyte dependent enzyme activation system has at least one enzyme selected from the group consisting of oxidoreductases, hydrolases, lyases, transferases, isomerases, and ligases.

**24.** An assay according to claim 23 wherein the enzyme is selected from the group consisting of peroxidases, oxidases, phosphatases, esterases and glycosidases.

**25.** An assay according to claim 24 wherein the enzyme is selected from the group consisting of horseradish peroxidase, glucose oxidase, alkaline phosphatase, and beta-galactosidase.

**26.** An assay according to claim 22 wherein the detectable label is selected from the group consisting of enzymes, radioactive isotopes, fluorogenic, chemiluminescent, or electrochemical materials or a member of a specific binding pair.

**27.** An assay according to claim 22 wherein the conjugate is reacted with a detectably labeled member of a specific binding pair.

**28.** An assay according to claim 22 wherein the conjugate is reacted with detectably labeled streptavidin.

**29.** An assay according to claim 22 wherein the conjugate is reacted with detectably labeled antibody.

**30.** An assay according to claim 22 wherein said assay is an immunoassay.

**31.** A compound which is 6-(phenoxy-(4'-azo-2''-carboxyethylphenyl))-hexanoyl-alkaline phosphatase.

**Claims for the following Contracting State : ES**

**1.** A method for the detection or quantitation of an analyte in an assay which comprises using an analyte dependent enzyme activation system comprising at least one enzyme to react with a conjugate consisting of a detectably labeled substrate specific for the enzyme system to form an activated conjugate which deposits substantially wherever at least one receptor for the activated conjugate is immobilized, said receptor not being reactive with the analyte dependent enzyme activation system, wherein deposited detectable labels either directly or indirectly generate a signal which can be detected or quantitated.

**2.** A method according to claim 1 wherein at least one enzyme of the analyte dependent enzyme activation system is selected from the group consisting of oxidoreductases, hydrolases, lyases, transferases, isomerases, and ligases.

**3.** A method according to claim 2 wherein the enzyme is selected from the group consisting of peroxidases, oxidases, phosphatases, esterases and glycosidases.

**4.** A method according to claim 3 wherein the enzyme is selected from the group consisting of horseradish peroxidase, glucose oxidase, alkaline phosphatase and beta-galactosidase, and preferably is horseradish peroxidase.

**5.** A method according to claim 4 wherein the conjugate is selected from the group consisting of biotin-tyramine, p-hydroxyphenylpropionylbiocytin, or fluorescein-tyramine.

**6.** A method according to claim 1 wherein the conjugate is reacted with detectably labeled antibody.

**7.** A method according to claim 1 wherein the conjugate is reacted with a detectably labeled member of a specific binding pair.

**8.** A method according to claim 1 wherein the conjugate is reacted with detectably labeled streptavidin.

**9.** A method according to claim 1 wherein the detectable label is selected from the group consisting of enzymes, radioactive isotopes, fluorogenic, chemiluminescent, or electrochemical materials or a member of a specific binding pair.

**10.** A method according to claim 1 wherein the conjugate is 6-(phenoxy-(4'-azo-2''-carboxyethylphenyl))-hexanoyl-alkaline phosphatase.

**11.** An assay for detecting or quantitating the presence or absence of an analyte in a sample which comprises

a) immobilizing the analyte;

b) reacting the product of step (a) with an analyte-dependent enzyme activation system;

c) reacting the product of step (b) with a conjugate consisting of a detectably labeled substrate to form an activated conjugate which deposits substantially wherever receptor for the activated conjugate is immobilized, said receptor not being reactive with the analyte dependent enzyme activation system; and

d) detecting or quantitating the presence or absence of the analyte in the sample.

**12.** An assay according to claim 11 wherein the analyte dependent enzyme activation system has at least one enzyme selected from the group consisting of oxidoreductases, hydrolases, lyases, transferases, isomerases, and ligases.

**13.** An assay according to claim 12 wherein the enzyme is selected from the group consisting of peroxidases, oxidases, phosphatases, esterases and glycosidases.

**14.** An assay according to claim 13 wherein the enzyme is selected from the group consisting of horseradish peroxidase, glucose oxidase, alkaline phosphatase, and beta-galactosidase.

**15.** An assay according to claim 11 wherein the detectable label is selected from the group consisting of enzymes, radioactive isotopes, fluorogenic, chemiluminescent, or electrochemical materials or a member of a specific binding pair.

**16.** An assay according to claim 14 wherein the enzyme is horseradish peroxidase and the conjugate is selected from the group consisting of biotin tyramine, p-hydroxyphenylpropionyl-biocytin or fluorescein-tyramine.

**17.** A method according to claim 11 wherein the conjugate is 6-(phenoxy-(4'-azo-2''-carboxyethylphenyl))-hexanoyl-alkaline phosphatase.

**18.** An assay according to claim 11 wherein the conjugate is reacted with a detectably labeled member of a specific binding pair.

**19.** An assay according to claim 11 wherein the conjugate is reacted with detectably labeled antibody.

**20.** An assay according to claim 11 wherein the conjugate is reacted with detectably labeled streptavidin.

**21.** An assay according to claim 11 wherein said assay is an immunoassay.

**22.** An assay for detecting or quantitating the presence or absence of an analyte in a sample which comprises
a) reacting an analyte dependent enzyme activation system with a conjugate consisting of a detectably labeled substrate to form an activated conjugate which deposits substantially wherever receptor for the activated conjugate is immobilized, said receptor not being reactive with the analyte dependent enzyme activation system; and
b) detecting or quantitating the presence or absence of the analyte in the sample.

**23.** An assay according to claim 22 wherein the analyte dependent enzyme activation system has at least one enzyme selected from the group consisting of oxidoreductases, hydrolases, lyases, transferases, isomerases, and ligases.

**24.** An assay according to claim 23 wherein the enzyme is selected from the group consisting of peroxidases, oxidases, phosphatases, esterases and glycosidases.

**25.** An assay according to claim 24 wherein the enzyme is selected from the group consisting of horseradish peroxidase, glucose oxidase, alkaline phosphatase, and beta-galactosidase.

**26.** An assay according to claim 22 wherein the detectable label is selected from the group consisting of enzymes, radioactive isotopes, fluorogenic, chemiluminescent, or electrochemical materials or a member of a specific binding pair.

**27.** An assay according to claim 22 wherein the conjugate is reacted with a detectably labeled member of a specific binding pair.

**28.** An assay according to claim 22 wherein the conjugate is reacted with detectably labeled streptavidin.

**29.** An assay according to claim 22 wherein the conjugate is reacted with detectably labeled antibody.

**30.** An assay according to claim 22 wherein said assay is an immunoassay.

**31.** Process for preparing 6-(phenoxy-(4'-azo-2''-carboxyethylphenyl))-hexanoyl-alkaline phosphatase, which comprises
1) dissolving (N-succinimidyl)-6-(phenoxy-(4'-azo-2''-carboxy-ethylphenyl))hexanoate in a minium volume of dimethylsulfoxide, and
2) adding said solution to a buffered aqueous solution of alkaline phosphatase.

## Patentansprüche
## Patentansprüche für folgende Vertragsstaaten : AT, BE, CH, DE, DK, FR, GB, IT, LI, LU, NL, SE

**1.** Verfahren zum Nachweis oder der mengenmäßigen Bestimmung eines Analyten in einem Test, welches das Verwenden eines analytabhängigen Enzymaktivierungssystems umfaßt, das wenigstens ein Enzym zum Umsetzen mit einem Konjugat umfaßt, welches aus einem nachweisbar markierten Substrat besteht, das auf das Enzymsystem spezifisch ist, unter Bilden eines aktivierten Konjugats, das sich im wesentlichen überall dort festsetzt, wo wenigstens ein Rezeptor für das aktivierte Konjugat immobilisiert ist, wobei der Rezeptor mit dem analytabhängigen Enzymaktivierungssystem, in welchem die abgeschiedenen Markierungen entweder direkt oder indirekt ein Signal erzeugen, das nachgewiesen oder mengenmäßig bestimmt werden kann, nicht reaktionsfähig ist.

**2.** Verfahren gemäß Anspruch 1, bei welchem wenigstens ein Enzym des analytabhängigen Enzymaktivierungssystems aus der Gruppe ausgewählt ist, die aus Oxidoreduktasen, Hydrolasen, Lyasen, Transferasen, Isomerasen und Ligasen besteht.

**3.** Verfahren gemäß Anspruch 2, bei welchem das Enzym aus der Gruppe ausgewählt ist, die aus Peroxidasen, Oxidasen, Phosphatasen, Esterasen und Glykosidasen besteht.

4. Verfahren gemäß Anspruch 3, bei welchem das Enzym aus der Gruppe ausgewählt ist, die aus Meerrettichperoxidase, Glukoseoxidase, alkalischer Phosphatase und beta-Galaktosidase besteht, und vorzugsweise Meerrettichperoxidase ist.

5. Verfahren gemäß Anspruch 4, bei welchem das Konjugat aus der Gruppe ausgewählt ist, die aus Biotin-Tyramin, p-Hydroxyphenylpropionylbiocytin oder Fluorescein-Tyramin besteht.

6. Verfahren gemäß Anspruch 1, bei welchem das Konjugat mit nachweisbar markiertem Antikörper umgesetzt wird.

7. Verfahren gemäß Anspruch 1, bei welchem das Konjugat mit einem nachweisbar markierten Mitglied eines spezifischen Bindungspaares umgesetzt wird.

8. Verfahren gemäß Anspruch 1, bei welchem das Konjugat mit nachweisbar markiertem Streptavidin umgesetzt wird.

9. Verfahren gemäß Anspruch 1, bei welchem die nachweisbare Markierung aus der Gruppe, die aus Enzymen, radioaktiven Isotopen, fluorogenen, chemilumineszierenden oder elektrochemischen Materialien besteht, oder einem Mitglied eines spezifischen Bindungspaares ausgewählt ist.

10. Verfahren gemäß Anspruch 1, bei welchem das Konjugat 6-(Phenoxy-(4'-azo-2''-carboxyethylphenyl))-hexanoyl-alkalische Phosphatase ist.

11. Test zum Nachweisen oder mengenmäßigen Bestimmen der Anwesenheit oder Abwesenheit eines Analyten in einer Probe, der
    a) das Immobilisieren des Analyten;
    b) das Umsetzen des Produkts aus Schritt (a) mit einem analytabhängigen Enzymaktivierungssystem;
    c) das Umsetzen des Produkts aus Schritt (b) mit einem aus einem nachweisbar markierten Substrat bestehenden Konjugat unter Bilden eines aktivierten Konjugats, das sich im wesentlichen überall dort festsetzt, wo ein Rezeptor für das aktivierte Konjugat immobilisiert ist, wobei der Rezeptor mit dem analytabhängigen Enzymaktivierungssystem nicht reaktionsfähig ist und
    d) das Nachweisen oder mengenmäßige Bestimmen der Anwesenheit oder Abwesenheit des Analyten in der Probe umfaßt.

12. Test gemäß Anspruch 11, bei welchem das analytabhängige Enzymaktivierungssystem wenigstens ein Enzym besitzt, das aus der Gruppe ausgewählt ist, die aus Oxidoreduktasen, Hydrolasen, Lyasen, Transferasen, Isomerasen und Ligasen besteht.

13. Test gemäß Anspruch 12, bei welchem das Enzym aus der Gruppe ausgewählt ist, die aus Peroxidasen, Oxidasen, Phosphatasen, Esterasen und Glykosidasen besteht.

14. Test gemäß Anspruch 13, bei welchem das Enzym aus der Gruppe ausgewählt ist, die aus Meerrettichperoxidase, Glukoseoxidase, alkalischer Phosphatase und beta-Galaktosidase besteht.

15. Test gemäß Anspruch 11, bei welchem die nachweisbare Markierung aus der Gruppe, die aus Enzymen, radioaktiven Isotopen, fluorogenen, chemilumineszierenden oder elektrochemischen Materialien besteht, oder einem Mitglied eines spezifischen Bindungspaares ausgewählt ist.

16. Test gemäß Anspruch 14, bei welchem das Enzym Meerrettichperoxidase ist und das Konjugat aus der Gruppe ausgewählt ist, die aus Biotin-Tyramin, p-Hydroxyphenylpropionylbiocytin oder Fluorescein-Tyramin besteht.

17. Verfahren gemäß Anspruch 11, bei welchem das Konjugat 6-(Phenoxy-(4'-azo-2''-carboxyethylphenyl))-hexanoyl-alkalische Phosphatase ist.

18. Test gemäß Anspruch 11, bei welchem das Konjugat mit einem nachweisbar markierten Mitglied eines spezifischen Bindungspaars umgesetzt wird.

19. Test gemäß Anspruch 11, bei welchem das Konjugat mit einem nachweisbar markierten Antikörper umgesetzt wird.

20. Test gemäß Anspruch 11, bei welchem das Konjugat mit nachweisbar markiertem Streptavidin umgesetzt wird.

21. Test gemäß Anspruch 11, bei welchem der Test ein Immunassay ist.

22. Test zum Nachweisen oder mengenmäßigen Bestimmen der Anwesenheit oder Abwesenheit eines Analyten in einer Probe, welcher
   a) das Umsetzen eines analytabhängigen Enzymaktivierungssystems mit einem aus einem nachweisbar markierten Substrat bestehenden Konjugat unter Bilden eines aktivierten Konjugats, welches sich im wesentlichen überall dort festsetzt, wo ein Rezeptor für das aktivierte Konjugat immobilisiert ist, wobei der Rezeptor mit dem analytabhängigen Enzymaktivierungssystem nicht reaktionsfähig ist, und
   b) das Nachweisen oder mengenmäßige Bestimmen der Anwesenheit oder Abwesenheit des Analyten in der Probe umfaßt.

23. Test gemäß Anspruch 22, bei welchem das analytabhängige Enzymaktivierungssystem wenigstens ein Enzym besitzt, das aus der Gruppe ausgewählt ist, die aus Oxidoreduktasen, Hydrolasen, Lyasen, Transferasen, Isomerasen und Ligasen besteht.

24. Test gemäß Anspruch 23, bei welchem das Enzym aus der Gruppe ausgewählt ist, die aus Peroxidasen, Oxidasen, Phosphatasen, Esterasen und Glykosidasen besteht.

25. Test gemäß Anspruch 24, bei welchem das Enzym aus der Gruppe ausgewählt ist, die aus Meerrettichperoxidase, Glukoseoxidase, alkalischer Phosphatase und beta-Galaktosidase besteht.

26. Test gemäß Anspruch 22, bei welchem die nachweisbare Markierung aus der Gruppe, die aus Enzymen, radioaktiven Isotopen, fluorogenen, chemilumineszierenden oder elektrochemischen Materialien besteht, oder einem Mitglied eines spezifischen Bindungspaares ausgewählt ist.

27. Test gemäß Anspruch 22, bei welchem das Konjugat mit einem nachweisbar markierten Mitglied eines spezifischen Bindungspaars umgesetzt wird.

28. Test gemäß Anspruch 22, bei welchem das Konjugat mit nachweisbar markiertem Streptavidin umgesetzt wird.

29. Test gemäß Anspruch 22, bei welchem das Konjugat mit einem nachweisbar markierten Antikörper umgesetzt wird.

30. Test gemäß Anspruch 22, bei welchem der Test ein Immunassay ist.

31. Verbindung, welche 6-(Phenoxy-(4'-azo-2''-carboxyethylphenyl))-hexanoyl-alkalische Phosphatase ist.

**Patentansprüche für folgende Vertragsstaaten : ES**

1. Verfahren zum Nachweis oder der mengenmäßigen Bestimmung eines Analyten in einem Test, welches das Verwenden eines analytabhängigen Enzymaktivierungssystems umfaßt, das wenigstens ein Enzym zum Umsetzen mit einem Konjugat umfaßt, welches aus einem nachweisbar markierten Substrat besteht, das auf das Enzymsystem spezifisch ist, unter Bilden eines aktivierten Konjugats, das sich im wesentlichen überall dort festsetzt, wo wenigstens ein Rezeptor für das aktivierte Konjugat immobilisiert ist, wobei der Rezeptor mit dem analytabhängigen Enzymaktivierungssystem, in welchem die abgeschiedenen Markierungen entweder direkt oder indirekt ein Signal erzeugen, das nachgewiesen oder mengenmäßig bestimmt werden kann, nicht reaktionsfähig ist.

2. Verfahren gemäß Anspruch 1, bei welchem wenigstens ein Enzym des analytabhängigen Enzymaktivierungssystems aus der Gruppe ausgewählt ist, die aus Oxidoreduktasen, Hydrolasen, Lyasen, Transferasen, Isomerasen und Ligasen besteht.

**3.** Verfahren gemäß Anspruch 2, bei welchem das Enzym aus der Gruppe ausgewählt ist, die aus Peroxidasen, Oxidasen, Phosphatasen, Esterasen und Glykosidasen besteht.

**4.** Verfahren gemäß Anspruch 3, bei welchem das Enzym aus der Gruppe ausgewählt ist, die aus Meerrettichperoxidase, Glukoseoxidase, alkalischer Phosphatase und beta-Galaktosidase besteht, und vorzugsweise Meerrettichperoxidase ist.

**5.** Verfahren gemäß Anspruch 4, bei welchem das Konjugat aus der Gruppe ausgewählt ist, die aus Biotin-Tyramin, p-Hydroxyphenylpropionylbiocytin oder Fluorescein-Tyramin besteht.

**6.** Verfahren gemäß Anspruch 1, bei welchem das Konjugat mit nachweisbar markiertem Antikörper umgesetzt wird.

**7.** Verfahren gemäß Anspruch 1, bei welchem das Konjugat mit einem nachweisbar markierten Mitglied eines spezifischen Bindungspaares umgesetzt wird.

**8.** Verfahren gemäß Anspruch 1, bei welchem das Konjugat mit nachweisbar markiertem Streptavidin umgesetzt wird.

**9.** Verfahren gemäß Anspruch 1, bei welchem die nachweisbare Markierung aus der Gruppe, die aus Enzymen, radioaktiven Isotopen, fluorogenen, chemilumineszierenden oder elektrochemischen Materialien besteht, oder einem Mitglied eines spezifischen Bindungspaares ausgewählt ist.

**10.** Verfahren gemäß Anspruch 1, bei welchem das Konjugat 6-(Phenoxy-(4'-azo-2''-carboxyethylphenyl))-hexanoyl-alkalische Phosphatase ist.

**11.** Test zum Nachweisen oder mengenmäßigen Bestimmen der Anwesenheit oder Abwesenheit eines Analyten in einer Probe, der

a) das Immobilisieren des Analyten;

b) das Umsetzen des Produkts aus Schritt (a) mit einem analytabhängigen Enzymaktivierungssystem;

c) das Umsetzen des Produkts aus Schritt (b) mit einem aus einem nachweisbar markierten Substrat bestehenden Konjugat unter Bilden eines aktivierten Konjugats, das sich im wesentlichen überall dort festsetzt, wo ein Rezeptor für das aktivierte Konjugat immobilisiert ist, wobei der Rezeptor mit dem analytabhängigen Enzymaktivierungssystem nicht reaktionsfähig ist und

d) das Nachweisen oder mengenmäßige Bestimmen der Anwesenheit oder Abwesenheit des Analyten in der Probe umfaßt.

**12.** Test gemäß Anspruch 11, bei welchem das analytabhängige Enzymaktivierungssystem wenigstens ein Enzym besitzt, das aus der Gruppe ausgewählt ist, die aus Oxidoreduktasen, Hydrolasen, Lyasen, Transferasen, Isomerasen und Ligasen besteht.

**13.** Test gemäß Anspruch 12, bei welchem das Enzym aus der Gruppe ausgewählt ist, die aus Peroxidasen, Oxidasen, Phosphatasen, Esterasen und Glykosidasen besteht.

**14.** Test gemäß Anspruch 13, bei welchem das Enzym aus der Gruppe ausgewählt ist, die aus Meerrettichperoxidase, Glukoseoxidase, alkalischer Phosphatase und beta-Galaktosidase besteht.

**15.** Test gemäß Anspruch 11, bei welchem die nachweisbare Markierung aus der Gruppe, die aus Enzymen, radioaktiven Isotopen, fluorogenen, chemilumineszierenden oder elektrochemischen Materialien besteht, oder einem Mitglied eines spezifischen Bindungspaares ausgewählt ist.

**16.** Test gemäß Anspruch 14, bei welchem das Enzym Meerrettichperoxidase ist und das Konjugat aus der Gruppe ausgewählt ist, die aus Biotin-Tyramin, p-Hydroxyphenylpropionylbiocytin oder Fluorescein-Tyramin besteht.

**17.** Verfahren gemäß Anspruch 11, bei welchem das Konjugat 6-(Phenoxy-(4'-azo-2''-carboxyethylphenyl))-hexanoyl-alkalische Phosphatase ist.

**18.** Test gemäß Anspruch 11, bei welchem das Konjugat mit einem nachweisbar markierten Mitglied eines spezifischen Bindungspaars umgesetzt wird.

**19.** Test gemäß Anspruch 11, bei welchem das Konjugat mit einem nachweisbar markierten Antikörper umgesetzt wird.

**20.** Test gemäß Anspruch 11, bei welchem das Konjugat mit nachweisbar markiertem Streptavidin umgesetzt wird.

**21.** Test gemäß Anspruch 11, bei welchem der Test ein Immunassay ist.

**22.** Test zum Nachweisen oder mengenmäßigen Bestimmen der Anwesenheit oder Abwesenheit eines Analyten in einer Probe, welcher

a) das Umsetzen eines analytabhängigen Enzymaktivierungssystems mit einem aus einem nachweisbar markierten Substrat bestehenden Konjugat unter Bilden eines aktivierten Konjugats, welches sich im wesentlichen überall dort festsetzt, wo ein Rezeptor für das aktivierte Konjugat immobilisiert ist, wobei der Rezeptor mit dem analytabhängigen Enzymaktivierungssystem nicht reaktionsfähig ist, und

b) das Nachweisen oder mengenmäßige Bestimmen der Anwesenheit oder Abwesenheit des Analyten in der Probe umfaßt.

**23.** Test gemäß Anspruch 22, bei welchem das analytabhängige Enzymaktivierungssystem wenigstens ein Enzym besitzt, das aus der Gruppe ausgewählt ist, die aus Oxidoreduktasen, Hydrolasen, Lyasen, Transferasen, Isomerasen und Ligasen besteht.

**24.** Test gemäß Anspruch 23, bei welchem das Enzym aus der Gruppe ausgewählt ist, die aus Peroxidasen, Oxidasen, Phosphatasen, Esterasen und Glykosidasen besteht.

**25.** Test gemäß Anspruch 24, bei welchem das Enzym aus der Gruppe ausgewählt ist, die aus Meerrettichperoxidase, Glukoseoxidase, alkalischer Phosphatase und beta-Galaktosidase besteht.

**26.** Test gemäß Anspruch 22, bei welchem die nachweisbare Markierung aus der Gruppe, die aus Enzymen, radioaktiven Isotopen, fluorogenen, chemilumineszierenden oder elektrochemischen Materialien besteht, oder einem Mitglied eines spezifischen Bindungspaares ausgewählt ist.

**27.** Test gemäß Anspruch 22, bei welchem das Konjugat mit einem nachweisbar markierten Mitglied eines spezifischen Bindungspaars umgesetzt wird.

**28.** Test gemäß Anspruch 22, bei welchem das Konjugat mit nachweisbar markiertem Streptavidin umgesetzt wird.

**29.** Test gemäß Anspruch 22, bei welchem das Konjugat mit einem nachweisbar markierten Antikörper umgesetzt wird.

**30.** Test gemäß Anspruch 22, bei welchem der Test ein Immunassay ist.

**31.** Verfahren zum Herstellen von 6-(Phenoxy-(4'-azo-2''-carboxyethylphenyl))-hexanoyl-alkalischer Phosphatase, welches

1) das Lösen von (N-Succinimidyl)-6-(phenoxy-(4'-azo-2''-carboxyethylphenyl))-hexanoat im Mindestvolumen Dimethylsulfoxid und

2) das Hinzusetzen der Lösung einer gepufferten, wäßrigen Lösung von alkalischer Phosphatase umfaßt.

## Revendications

**Revendications pour les Etats contractants suivants : AT, BE, CH, DE, DK, FR, GB, IT, LI, LU, NL, SE**

1. Procédé pour la détection ou la détermination quantitative d'un analyte dans un essai, qui comprend l'utilisation d'un système d'activation d'enzymes dépendant de l'analyte comprenant au moins une enzyme destinée à réagir avec un conjugué consistant en un substrat marqué de façon détectable et qui est spécifique du système d'enzyme en vue de former un conjugué activé qui se dépose pratiquement en tout endroit où au moins un récepteur pour le conjugué activé est immobilisé, ce récepteur ne réagissant pas réactif avec le système d'activation d'enzymes dépendant de l'analyte, et, dans ce procédé, les marqueurs détectables déposés génèrant soit directement soit indirectement un signal qui peut être détecté ou quantifié.

2. Procédé selon la revendication 1, dans lequel au moins une enzyme du système d'activation d'enzymes dépendant de l'analyte est choisie dans le groupe consistant en oxydoréductases, hydrolases, lyases, transférases, isomérases et ligases.

3. Procédé selon la revendication 2, dans lequel l'enzyme est choisie dans le groupe consistant en peroxydases, oxydases, phosphatases, estérases et glycosidases.

4. Procédé selon la revendication 3, dans lequel l'enzyme est choisie dans le groupe consistant en peroxydase de raifort, glucose oxydase, phosphatase alcaline et béta-galactosidase, et est de préférence une peroxydase de raifort.

5. Procédé selon la revendication 4, dans lequel le conjugué est choisi dans le groupe consistant en biotine-tyramine, p-hydroxyphénylpropionylbiocytine ou fluorescéine-tyramine.

6. Procédé selon la revendication 1, dans lequel le conjugué est traité avec un anticorps marqué de façon à pouvoir être détecté.

7. Procédé selon la revendication 1, dans lequel le conjugué est traité par l'un des membres se liant de façon spécifique et marqué de façon à pouvoir être détectée.

8. Procédé selon la revendication 1, dans lequel le conjugué est traité avec de la streptavidine marquée de façon à pouvoir être détectée.

9. Procédé selon la revendication 1, dans lequel le marqueur détectable est choisi dans le groupe consistant en enzymes, isotopes radioactifs, matières fluorogènes, chimioluminescentes ou électrochimiques, ou un membre d'une paire susceptible de se lier de féion specifique.

10. Procédé selon la revendication 1, dans lequel le conjugué est la 6-[phénoxy-(4'-azo-2''-carboxyéthyl-phényl)]-hexanoyl-pho sphatase alcaline.

11. Un test pour la détection ou la détermination quantitative de la présence ou de l'absence d'un analyte dans un échantillons qui comprend :
    (a) l'immobilisation de l'analyte;
    (b) la réaction du produit de l'étape (a) avec un système d'activation d'enzymes dépendant de l'analyte;
    (c) la réaction du produit de l'étape (b) avec un conjugué consistant en un substrat marqué de façon détectable en vue de former un conjugué activé qui se dépose pratiquement en tout endroit où un récepteur pour le conjugué activé est immobilisé, ce récepteur ne réagissant pas avec le système d'activation d'enzymes dépendant de l'analyte; et
    (d) la détection ou la détermination quantitative de la présence ou de l'absence de l'analyte dans l'échantillon.

12. Essai selon la revendication 11, dans lequel le système d'activation d'enzymes dépendant de l'analyte comprend au moins une enzyme choisie dans le groupe consistant en oxydoréductases, hydrolases, lyases, transférases, isomérases et ligases.

**13.** Essai selon la revendication 12, dans lequel l'enzyme est choisie dans le groupe consistant en peroxydases, oxydases, phosphatases, estérases et glycosidases.

**14.** Essai selon la revendication 13, dans lequel l'enzyme est choisie dans le groupe consistant en peroxydase de raifort, glucose oxydase, phosphatase alcaline et béta-galactosidase.

**15.** Essai selon la revendication 11, dans lequel le marqueur détectable est choisi dans le groupe consistant en enzymes, isotopes radioactifs, matières fluorogènes, chimioluminescentes ou électrochimiques, ou un membre d'une paire susceptible de se lier de façon specifique.

**16.** Essai selon la revendication 14, dans lequel l'enzyme est une peroxydase de raifort et le conjugué est choisi dans le groupe consistant en biotine-tyramine, p-hydroxyphénylpropionyl-biocytine ou fluorescéine-tyramine.

**17.** Essai selon la revendication 11, dans lequel le conjugué est la 6-[phénoxy-(4'-azo-2''-carboxyéthylphényl)]-hexanoyl-pho sphatase alcaline.

**18.** Essai selon la revendication 11, dans lequel le conjugué est traité avec un membre d'une paire susceptible de se lier de façon spécifique, qui est marqué de façon à pouvoir être détecté.

**19.** Essai selon la revendication 11, dans lequel le conjugué est traité avec un anticorps marqué de façon à pouvoir être détecté.

**20.** Essai selon la revendication 11, dans lequel le conjugué est traité avec de la streptavidine marquée de façon à pouvoir être détectée.

**21.** Essai selon la revendication 11, dans lequel ce test est un test immunologique.

**22.** Un test pour la détection ou la détermination quantitative de la présence ou de l'absence d'un analyte dans un échantillon, qui comprend :

(a) la réaction d'un système d'activation d'enzymes dépendant de l'analyte avec un conjugué consistant en un substrat marqué de façon détectable pour former un conjugué activé qui se dépose pratiquement en tout endroit où un récepteur pour le conjugué activé est immobilisé, ce récepteur ne réagissant pas avec le système d'activation d'enzymes dépendant de l'analyte; et

(b) la détection ou la détermination quantitative de la présence ou de l'absence de l'analyte dans l'échantillon.

**23.** Essai selon la revendication 22, dans lequel le système d'activation d'enzymes dépendant de l'analyte comprend au moins une enzyme choisie dans le groupe consistant en oxydoréductases, hydrolases, lyases, transférases, isomérases et ligases.

**24.** Essai selon la revendication 23, dans lequel l'enzyme est choisie dans le groupe consistant en peroxydases, oxydases, phosphatases, estérases et glycosidases.

**25.** Essai selon la revendication 24, dans lequel l'enzyme est choisie dans le groupe consistant en peroxydase de raifort, glucose oxydase, phosphatase alcaline et béta-galactosidase.

**26.** Essai selon la revendication 22, dans lequel le marqueur détectable est choisi dans le groupe consistant en enzymes, isotopes radioactifs, matières fluorogènes, chimioluminescentes ou électrochimiques, ou un membre d'une paire de liaison specifique.

**27.** Essai selon la revendication 22, dans lequel le conjugué est traité avec un membre marqué de façon à pouvoir être détecté d'une paire susceptible de se lier de façon spécifique.

**28.** Essai selon la revendication 11, dans lequel le conjugué est traité avec de la streptavidine marquée de façon à pouvoir être détectée.

**29.** Essai selon la revendication 22, dans lequel le conjugué est traité avec un anticorps marqué de façon à pouvoir être détecté.

**30.** Essai selon la revendication 22, dans lequel ce test est un test immunologique.

**31.** Composé qui est la 6-[phénoxy-(4'-azo-2''-carboxyéthyl-phényl)]-hexanoyl-ph osphatase alcaline.

**Revendications pour l'Etat contractant suivant : ES**

**1.** Procédé pour la détection ou la détermination quantitative d'un analyte dans un test, qui comprend l'utilisation d'un système d'activation d'enzymes dépendant de l'analyte comprenant au moins une enzyme destinée à réagir avec un conjugué consistant en un substrat marqué de façon détectable et qui est spécifique du système d'enzyme en vue de former un conjugué activé qui se dépose pratiquement en tout endroit où au moins un récepteur pour le conjugué activé est immobilisé, ce récepteur ne réagissant pas avec le système d'activation d'enzymes dépendant de l'analyte, et, dans ce procédé, les marqueurs détectables déposés génèrant soit directement soit indirectement un signal qui peut être détecté ou quantifié.

**2.** Procédé selon la revendication 1, dans lequel au moins une enzyme du système d'activation d'enzymes dépendant de l'analyte est choisie dans le groupe consistant en oxydoréductases, hydrolases, lyases, transférases, isomérases et ligases.

**3.** Procédé selon la revendication 2, dans lequel l'enzyme est choisie dans le groupe consistant en peroxydases, oxydases, phosphatases, estérases et glycosidases.

**4.** Procédé selon la revendication 3, dans lequel l'enzyme est choisie dans le groupe consistant en peroxydase de raifort, glucose oxydase, phosphatase alcaline et béta-galactosidase, et est de préférence une peroxydase de raifort.

**5.** Procédé selon la revendication 4, dans lequel le conjugué est choisi dans le groupe consistant en biotine-tyramine, p-hydroxyphénylpropionylbiocytine ou fluorescéine-tyramine.

**6.** Procédé selon la revendication 1, dans lequel le conjugué est traité avec un anticorps marqué de façon à pouvoir être détecté.

**7.** Procédé selon la revendication 1, dans lequel le conjugué est traité par l'un des membres se liant de façon spécifique et marqué de façon à pouvoir être détecté.

**8.** Procédé selon la revendication 1, dans lequel le conjugué est traité avec de la streptavidine marquée de façon à pouvoir être détectée.

**9.** Procédé selon la revendication 1, dans lequel le marqueur détectable est choisi dans le groupe consistant en enzymes, isotopes radioactifs, matières fluorogènes, chimioluminescentes ou électrochimiques, ou un membre d'une paire susceptible de se lier de façon specifique.

**10.** Procédé selon la revendication 1, dans lequel le conjugué est la 6-[phénoxy-(4'-azo-2''-carboxyéthyl-phényl)]-hexanoyl-pho sphatase alcaline.

**11.** Un test permettant la détection ou la détermination quantitative de la présence ou de l'absence d'un analyte dans un échantillon, qui comprend :

(a) l'immobilisation de l'analyte;

(b) la réaction du produit de l'étape (a) avec un système d'activation d'enzymes dépendant de l'analyte;

(c) la réaction du produit de l'étape (b) avec un conjugué consistant en un substrat marqué de façon détectable en vue de former un conjugué activé qui se dépose pratiquement en tout endroit où un récepteur pour le conjugué activé est immobilisé, ce récepteur ne réagissant pas avec le système d'activation d'enzymes dépendant de l'analyte; et

(d) la détection ou la détermination quantitative de la présence ou de l'absence de l'analyte dans l'échantillon.

**12.** Essai selon la revendication 11, dans lequel le système d'activation d'enzymes dépendant de l'analyte comprend au moins une enzyme choisie dans le groupe consistant en oxydoréductases, hydrolases, lyases, transférases, isomérases et ligases.

**13.** Essai selon la revendication 12, dans lequel l'enzyme est choisie dans le groupe consistant en peroxydases, oxydases, phosphatases, estérases et glycosidases.

**14.** Essai selon la revendication 13, dans lequel l'enzyme est choisie dans le groupe consistant en peroxydase du raifort, glucose oxydase, phosphatase alcaline et béta-galactosidase.

**15.** Essai selon la revendication 11, dans lequel le marqueur détectable est choisi dans le groupe consistant en enzymes, isotopes radioactifs, matières fluorogènes, chimioluminescentes ou électrochimiques, ou un membre d'une paire susceptible de se fier de façon spécifique.

**16.** Essai selon la revendication 14, dans lequel l'enzyme est une peroxydase de raifort et le conjugué est choisi dans le groupe consistant en biotine-tyramine, p-hydroxyphénylpropionyl-biocytine ou fluorescéine-tyramine.

**17.** Essai selon la revendication 11, dans lequel le conjugué est la 6-[phénoxy-(4'-azo-2''-carboxyéthylphényl)]-hexanoyl-pho sphatase alcaline.

**18.** Essai selon la revendication 11, dans lequel le conjugué est traité avec un membre d'une paire susceptible de se lier de façon spécifique, qui est marqué de façon à pouvoir être détecté.

**19.** Essai selon la revendication 11, dans lequel le conjugué est traité avec un anticorps marqué de façon à pouvoir être détecté.

**20.** Essai selon la revendication 11, dans lequel le conjugué est traité avec de la streptavidine marquée de façon à pouvoir être détectée.

**21.** Essai selon la revendication 11, dans lequel ce test est un test immunologique.

**22.** Un test pour la détection ou la détermination quantitative de la présence ou de l'absence d'un analyte dans un échantillon, qui comprend :

(a) la réaction d'un système d'activation d'enzymes dépendant de l'analyte avec un conjugué consistant en un substrat marqué de façon détectable pour former un conjugué activé qui se dépose pratiquement en tout endroit où un récepteur pour le conjugué activé est immobilisé, ce récepteur ne réagissant pas avec le système d'activation d'enzymes dépendant de l'analyte; et

(b) la détection ou la détermination quantitative de la présence ou de l'absence de l'analyte dans l'échantillon.

**23.** Essai selon la revendication 22, dans lequel le système d'activation d'enzymes dépendant de l'analyte comprend au moins une enzyme choisie dans le groupe consistant en oxydoréductases, hydrolases, lyases, transférases, isomérases et ligases.

**24.** Essai selon la revendication 23, dans lequel l'enzyme est choisie dans le groupe consistant en peroxydases, oxydases, phosphatases, estérases et glycosidases.

**25.** Essai selon la revendication 24, dans lequel l'enzyme est choisie dans le groupe consistant en peroxydase de raifort, glucose oxydase, phosphatase alcaline et béta-galactosidase.

**26.** Essai selon la revendication 22, dans lequel le marqueur détectable est choisi dans le groupe consistant en enzymes, isotopes radioactifs, matières fluorogènes, chimioluminescentes ou électrochimiques, ou un membre d'une paire de liaison specifique.

**27.** Essai selon la revendication 22, dans lequel le conjugué est traité avec un membre marqué de façon à pouvoir être détecté d'une paire susceptible de se lier de façon spécifique.

**28.** Essai selon la revendication 11, dans lequel le conjugué est traité avec de la streptavidine marquée de façon à pouvoir être détectée.

**29.** Essai selon la revendication 22, dans lequel le conjugué est traité avec un anticorps marqué de façon à pouvoir être détecté.

**30.** Essai selon la revendication 22, dans lequel ce test est un test immunologique.

**31.** Procédé pour préparer la 6-[phénoxy-(4'-azo-2''-carboxyéthylphényl)]-hexanoyl-pho sphatase alcaline, qui comprend:
(1) la dissolution de 6-[phénoxy-(4'-azo-2''-carboxyéthylphényl)]-hexanoate de N-succinimidyle dans un volume minimum de diméthylsulfoxyde, et
(2) l'addition de cette solution à une solution aqueuse tamponnée de phosphatase alcaline.

FIG.1

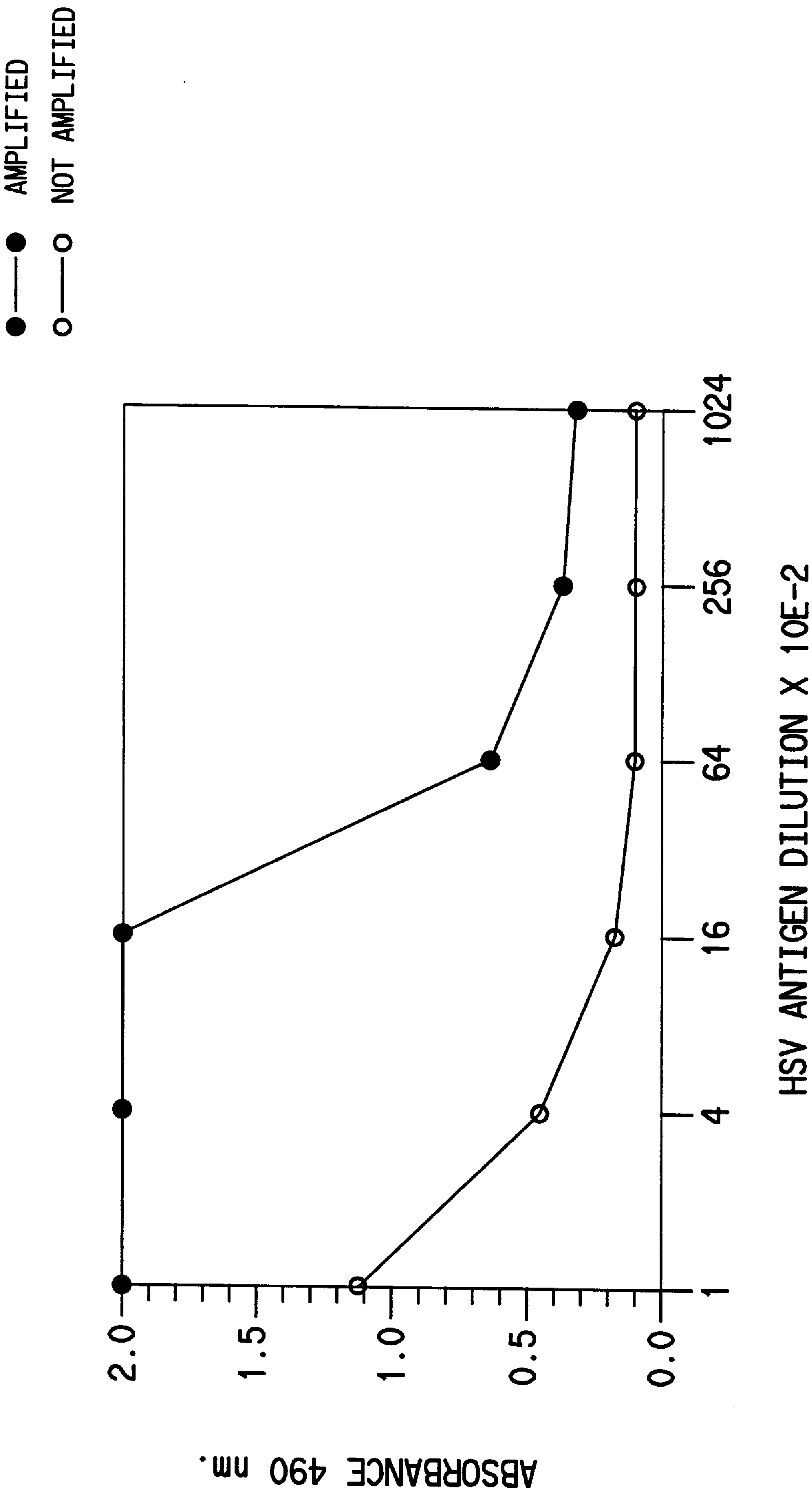
AMPLIFIED
NOT AMPLIFIED
ABSORBANCE 490 nm.
2.0
1.5
1.0
0.5
0.0
1
4
16
64
256
1024
HSV ANTIGEN DILUTION X 10E-2

FIG.2

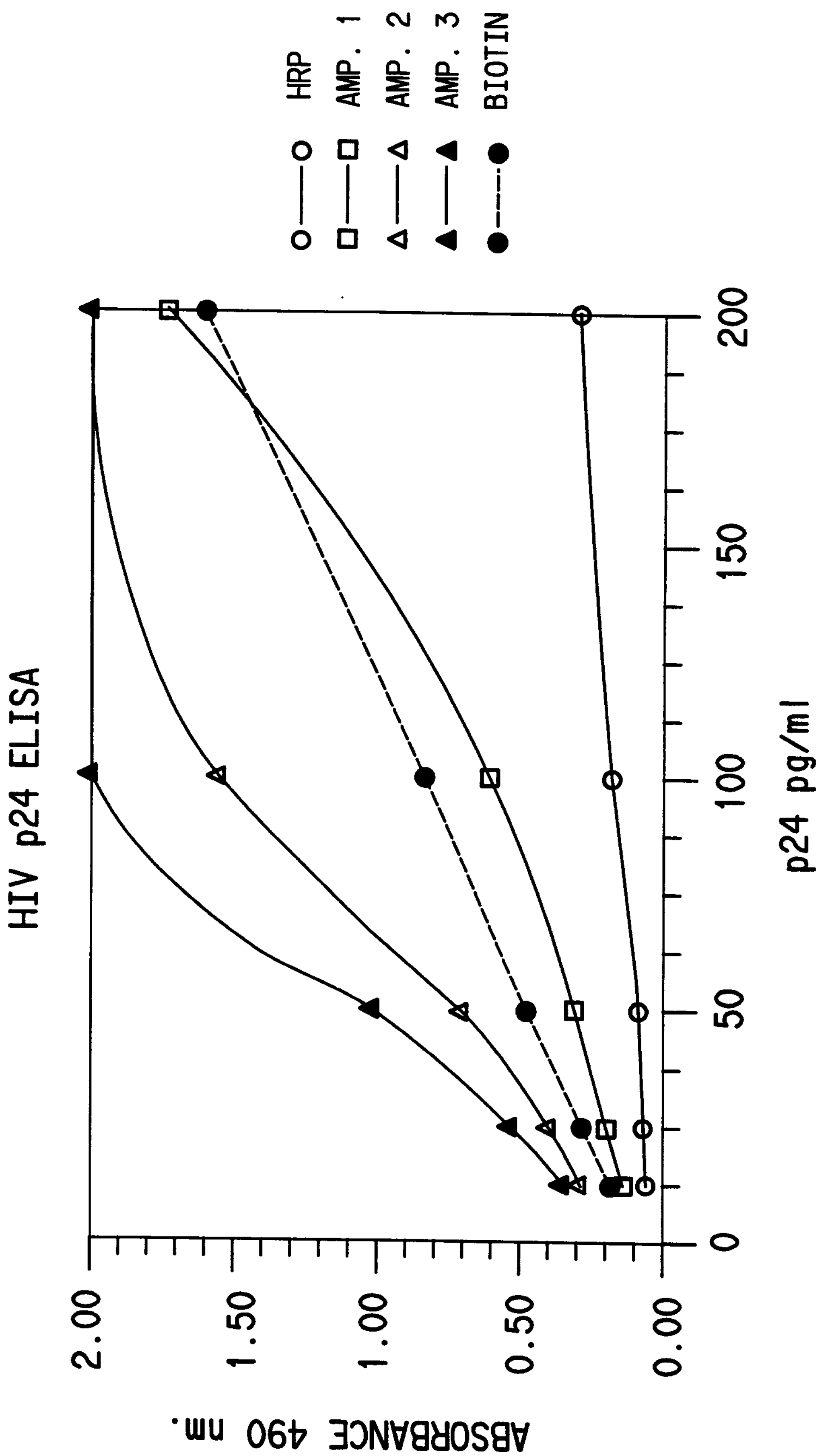
HIV p24 ELISA
HRP
AMP. 1
AMP. 2
AMP. 3
BIOTIN
ABSORBANCE 490 nm.
2.00
1.50
1.00
0.50
0.00
0
50
100
150
200
p24 pg/ml

FIG.3

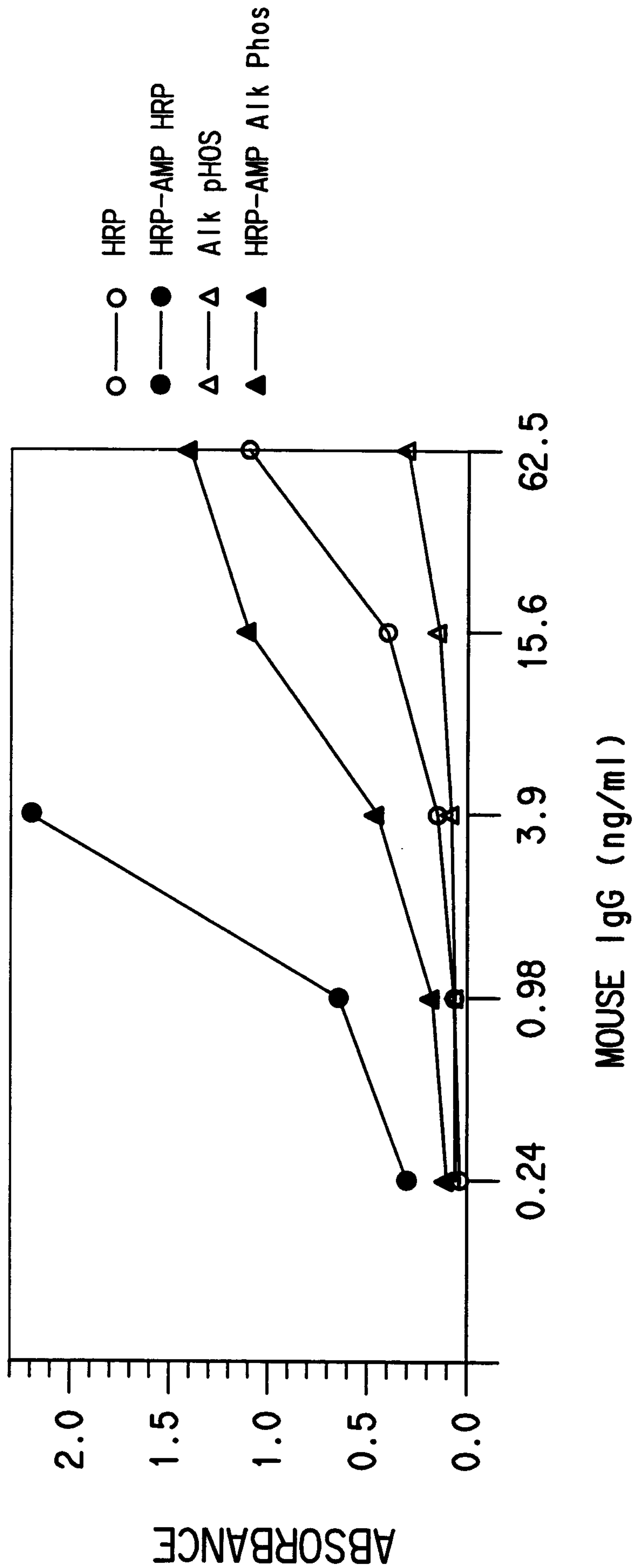
HRP
HRP-AMP HRP
Alk pHOS
HRP-AMP Alk Phos
ABSORBANCE
2.0
1.5
1.0
0.5
0.0
0.24
0.98
3.9
15.6
62.5
MOUSE IgG (ng/ml)

FIG.4A

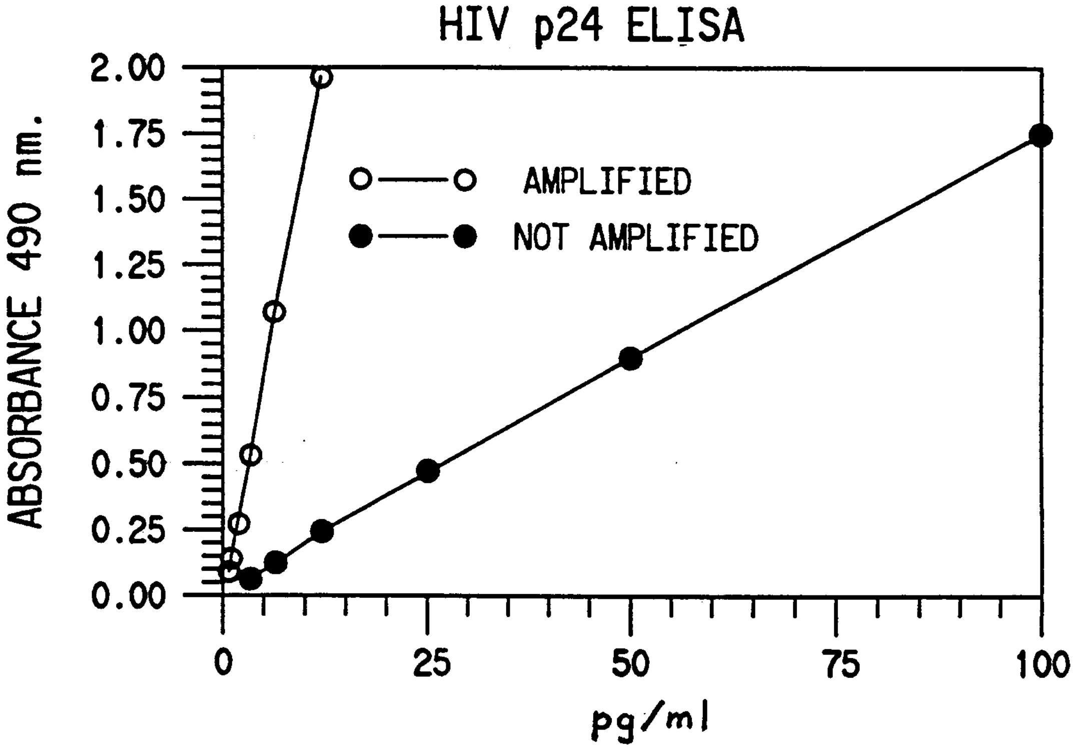
HIV p24 ELISA
ABSORBANCE 490 nm.
2.00
1.75
1.50
1.25
1.00
0.75
0.50
0.25
0.00
AMPLIFIED
NOT AMPLIFIED
0
25
50
75
100
pg/ml

FIG.4B

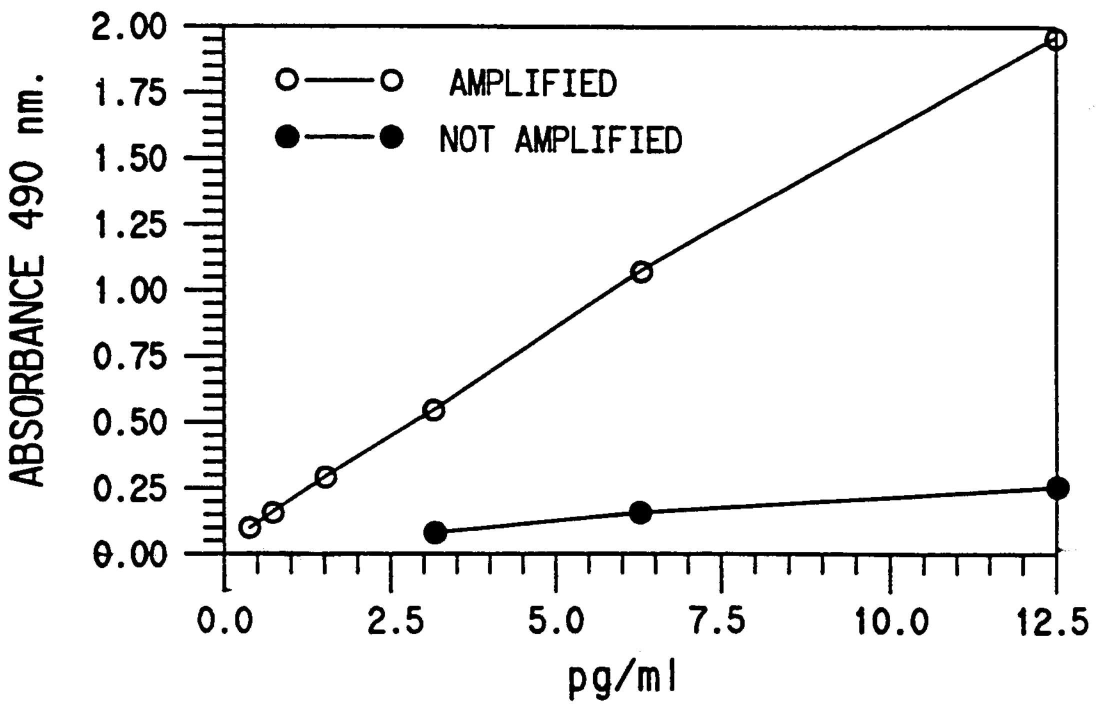
ABSORBANCE 490 nm.
2.00
1.75
1.50
1.25
1.00
0.75
0.50
0.25
0.00
AMPLIFIED
NOT AMPLIFIED
0.0
2.5
5.0
7.5
10.0
12.5
pg/ml

FIG.5

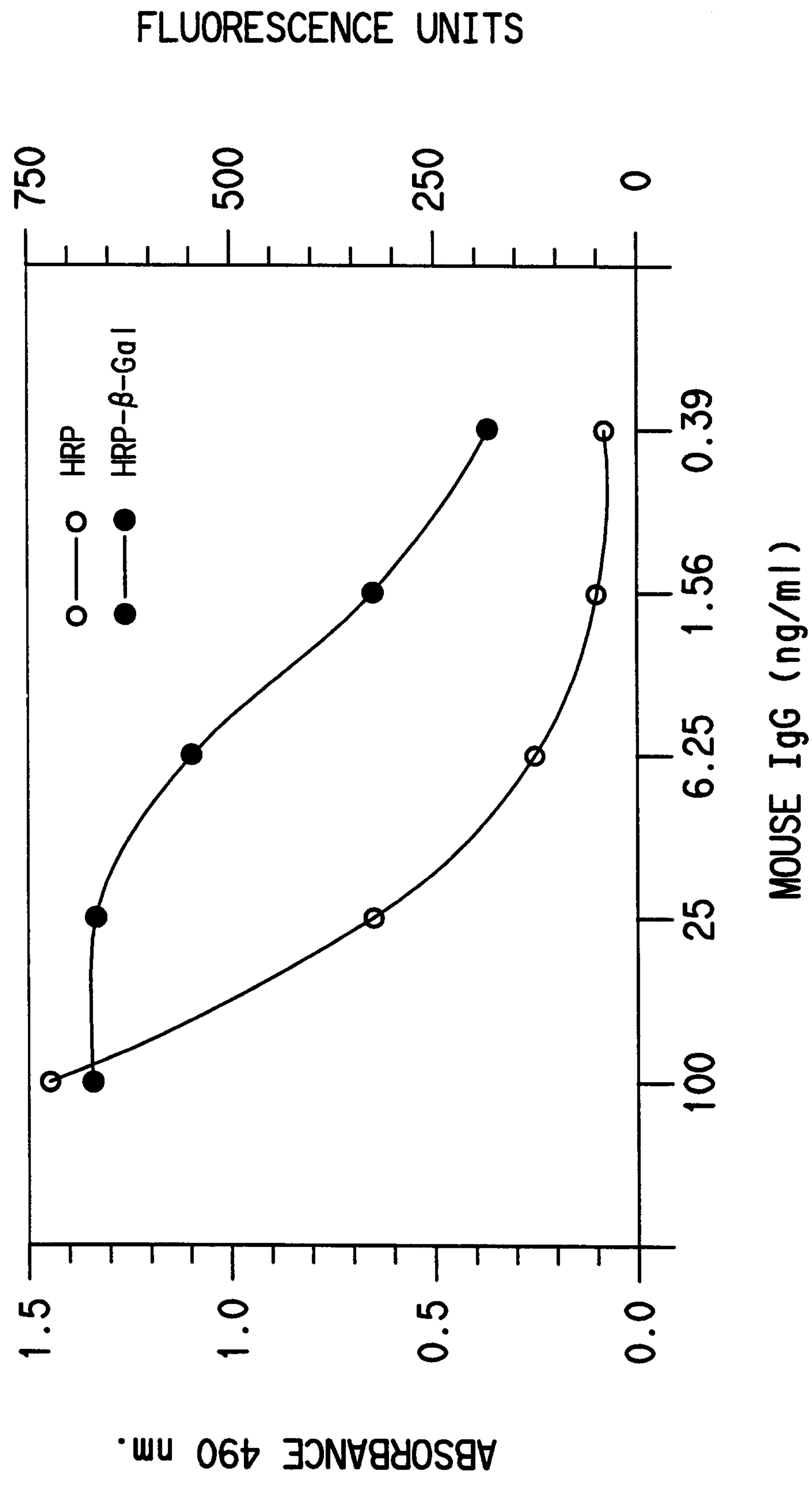
FLUORESCENCE UNITS
750
500
250
0
HRP
HRP-β-Gal
1.5
1.0
0.5
0.0
100
25
6.25
1.56
0.39
MOUSE IgG (ng/ml)
ABSORBANCE 490 nm.

# FIG.6

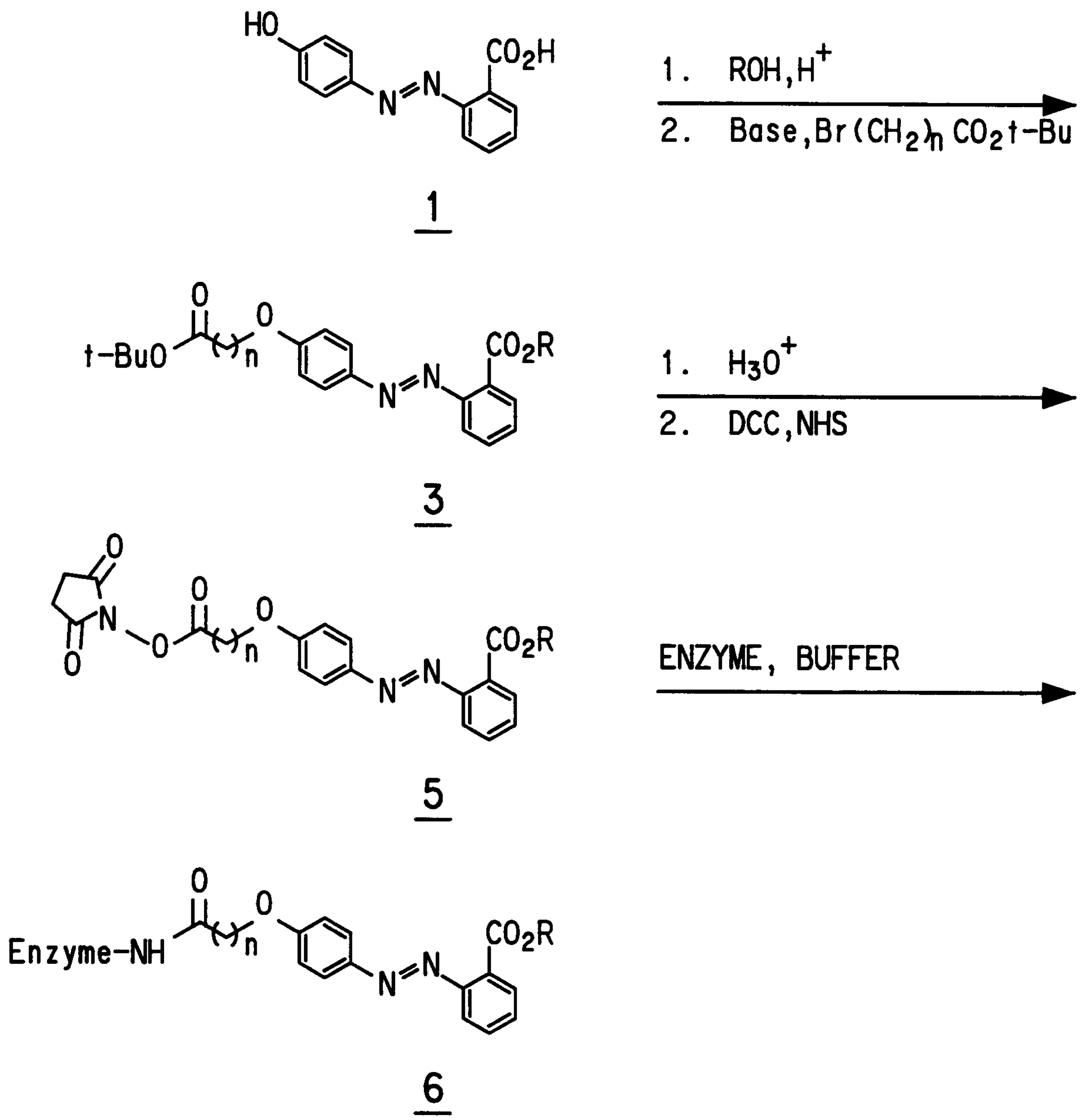


1

3

5

6

R = $CH_2CH_3$

n = 5

Enzyme = alkaline phosphatase

FIG.7

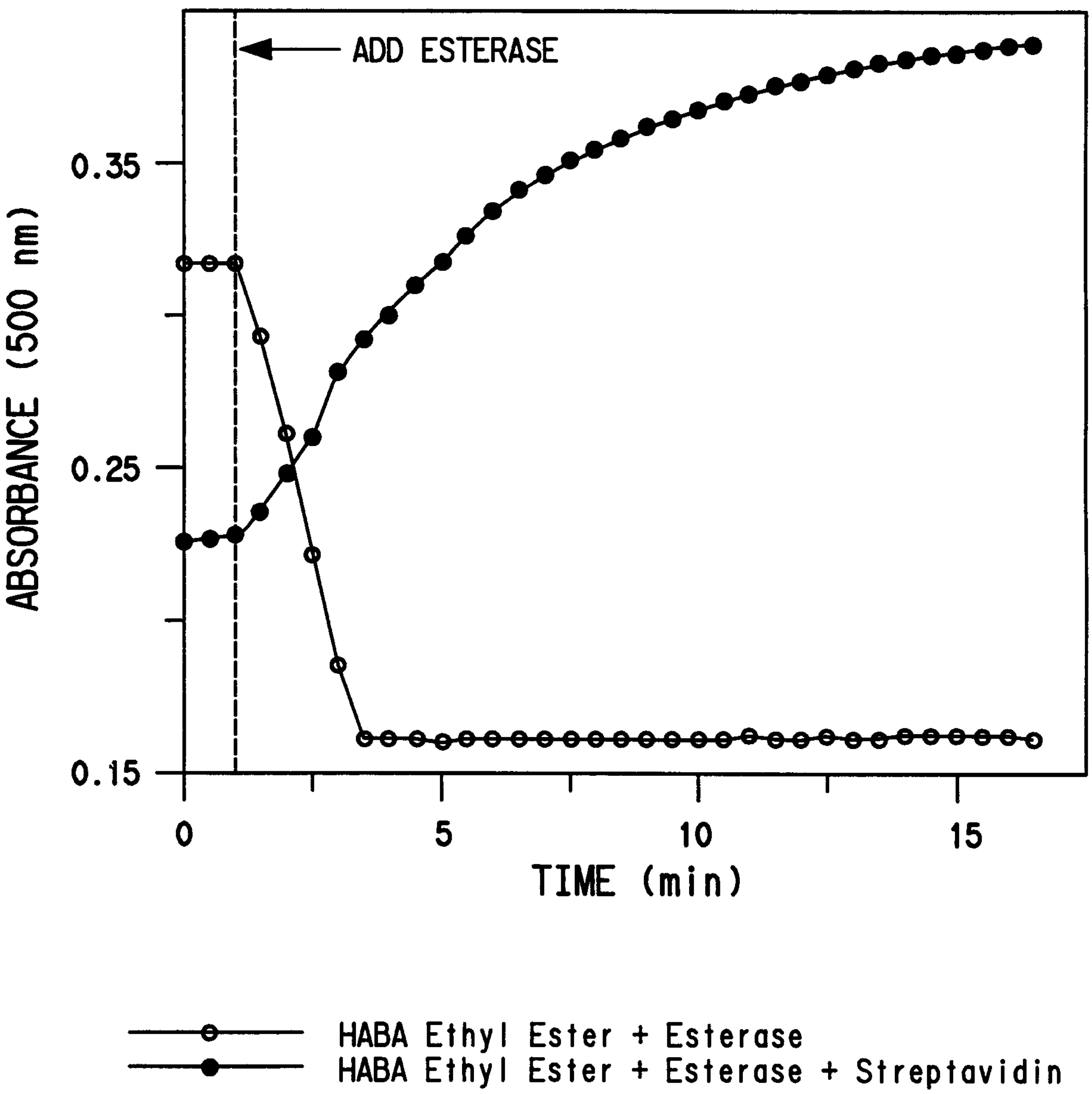
ADD ESTERASE
0.35
0.25
0.15
ABSORBANCE (500 nm)
0
5
10
15
TIME (min)
HABA Ethyl Ester + Esterase
HABA Ethyl Ester + Esterase + Streptavidin